(19) Europäisches Patentamt
European Patent Office
Office européen des brevets

(11) EP 1 281 399 A2

(12) **EUROPEAN PATENT APPLICATION**

(43) Date of publication:
**05.02.2003 Bulletin 2003/06**

(21) Application number: **02255156.8**

(22) Date of filing: **23.07.2002**

(51) Int Cl.[7]: **A61K 31/18**, A61K 31/27, A61K 31/325, A61K 31/64, A61P 17/00, A61P 17/08, A61P 17/10

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **01.08.2001 US 309336 P**

(71) Applicant: **WARNER-LAMBERT COMPANY**
**Morris Plains, New Jersey 07950 (US)**

(72) Inventor: **Homan, Reynold**
**Ann Arbor, Michigan 48103 (US)**

(74) Representative: **Hayles, James Richard et al**
**UK Patent Department,**
**Pfizer Limited,**
**Ramsgate Road**
**Sandwich, Kent CT13 9NJ (GB)**

(54) **Dual inhibitors of wax ester and cholesteryl ester synthesis for inhibiting sebum production**

(57) The present invention provides a method of inhibiting sebum production and treating sebaceous gland disorders comprising administering to a patient in need of said treatment an effective amount of a compound that inhibits both acyl-Coenzyme A:cholesteryl acyltransferase (ACAT), and acyl-Coenzyme A:fatty alcohol acyltransferase (AFAT), provided that the compound is not [(2,4,6-triisopropyl-phenyl)-acetyl]-sulfamic acid 2,6-diisopropylphenyl ester or a pharmaceutically acceptable salt or solvate thereof. The method of the invention is useful to treat sebaceous gland disorders caused or exacerbated by the overproduction of sebum, including oily skin, acne, seborrhea, perioral dermatitis, rosacea, and corticosteroid-induced acneiform lesions.

EP 1 281 399 A2

## Description

### FIELD OF THE INVENTION

The present invention relates to a method for using a compound that inhibits wax ester synthesis, and optionally also inhibits cholesteryl ester synthesis, to inhibit sebum production in a subject, thereby treating a sebaceous gland disorder characterized by the overproduction of sebum.

### BACKGROUND OF THE INVENTION

Dermal sebaceous glands are holocrine glands that secrete a mixture of lipids known as sebum. The term "sebum" refers a secretion of the sebaceous gland comprising, *inter alia,* triglycerides, free fatty acids, wax esters, squalene, cholesterol, and cholesteryl esters. Overproduction of sebum by sebaceous glands can lead to the appearance of oily skin, and can also contribute to the development of acne or other disorders, diseases or conditions of the skin. Agents to treat or prevent the overproduction of sebum have included those that tend to dry out the skin, including soaps, alcohol toners and astringents, but these are often not effective over the long term.

"Acne" is the term used to describe a group of dermatological disorders associated with a variety of etiologies. The group of acnes includes chloroacne, ciliaris, cystic, keratosa, and vulgaris. In its vulgaris form, acne occurs primarily in the face and trunk areas, affecting the appearance of the patient. Acne probably causes more mental pain and anguish to those afflicted by it than do many other diseases that, from a physical standpoint, may be much more severe.

The basic lesion common to the family of diseases referred to as acnes is the comedo or "blackhead" of a pilosebaceous follicle. The condition may be mild and transient with only a few blackheads that can readily be ejected by pressure and are of little concern, or may be severe, persistent, and very disfiguring with the more serious cases causing cystic lesions and frequently leaving permanent scarring.

What appears to occur in the development of acnes is an initial filling up of the follicle with a viscous, keratinous material. This impaction of horny material is the whitehead or blackhead. As a result of bacterial growth in these horny impactions, the follicle ruptures initiating the inflammatory phase of the disease, which takes the form of pustules, papules, cysts, and nodules. Although many different approaches have been used for the treatment of this affliction, none are universally effective and most possess undesirable side effects.

One of the commonly used methods for acne treatment is the use of "peeling," i.e., as astringent, agents for mild cases which cause exfoliation with the removal of some of the keratinous plugs. In the more serious cases where pustular or cystic lesions exist, the same are evacuated by incision and the contents expressed. Various other therapies have been employed, such as vaccine therapy, to assist in the control of chronic infection and increase the patient's resistance to *Staphylococci;* cortisone-type steroids; hormone therapy, which is applicable only for female patients who may be put on routine contraceptive regimen with estrogens; antibacterial therapy for the treatment of extensive pustular or cystic acne where the patient may be treated with tetracyclines, penicillin, erythromycin, or other of the antibacterial agents; and, in some instances, general surgical skin planing may be used. Systematic administration of hormones and antibacterials have been shown to have some therapeutic merit, but are unacceptable for chronic therapy.

The administration of large oral doses of vitamin A has been suggested as being beneficial in acne (see e. g., Straumford 1943, Northwest Med. 42:219-225), although other investigators have felt it to be ineffective (see, e.g., Anderson et al., 1963, Brit. Med. J. 2:294-296).

None of the common topical treatments has been found to be particularly effective against acne. Vitamin A acid has been applied topically (see, e.g., Stuttgen G., 1962, Dermatologica 124:65-80), achieving good results for those hyperkeratotic disorders that are responsive to high oral doses of vitamin A. However, the article reports no effective treatment for patients suffering from acne.

The treatment of acnes with isotretinoin and etretinate is described by Goldstein J.A. et al., 1982, J. Am. Acad. Dermatol. 6:760-765. Treatment of acnes with various potential therapeutic entities is discussed in "Evaluation of Potential Therapeutic Entities for the Treatment of Acne" Pharmacology of Retinoids in the Skin. Pharmacol. Skin 1989, Reichert and Shroot, eds, Karger, Basel, 3:104-122.

The effects of 13-cis-retinoic acid on the hamster Meibomian gland are discussed by Lambert R.W. et al., 1989, J. Invest. Derm., 93(2):321-325, while the effects of retinoids on psoriasis are discussed by Lowe N.J. et al., 1989, in "Systemic Retinoids in Psoriasis: Comparative Efficacy and Toxicity," Pharmacology of Retinoids in the Skin. 1989, Pharmacol. Skin, Reichert and Shroot eds, Karger, Basel, 3:104-122.

U.S. Patent No. 3,729,568 to Kligman, issued April 24, 1973, describes the use of vitamin A acid (retinoic acid or tretinoin) in the treatment of acne vulgaris. International Patent Publication WO 93/06086 by Pfizer Inc. describes the use of vitamin A acid derivatives in the treatment of skin diseases including acne. U.S. Patent No.4,703,110 to Shudo, issued October 27, 1987, describes the use of para-substituted benzoic acid derivatives in the treatment of dermatological disorders including cystic acne. U.S. Patent No.4,927,928 to Shroot et al., issued May 22, 1990, describes the use of benzamido compounds in the treatment of dermatological diseases having an inflammatory and/or immunoallergic component, including acne vulgaris, senile acne, and medicinal or professional acne. U.S. Patent No. 6,235,016 to Stewart, issued May 22, 2001, even describes the use of pulsed laser light to reduce the rate of production of sebum.

Compounds that inhibit acyl-coenzyme A:cholesteryl acyltransferase are known as ACAT inhibitors. Systemically administered ACAT inhibitors are useful in lowering serum cholesterol levels. ACAT inhibitors and methods for preparing them are taught in numerous U.S. patents and foreign patent publications, including U.S. Patent No. 4,716,175 to Hoefle et al, issued December 29, 1987, and U.S. Patent No. 5,633,287 to Lee et al., among many others.

U.S. Patent No. 6,133,326 to Mayne, issued October 17, 2000, describes the use of ACAT inhibitors to decrease sebum production and to treat sebaceous gland disorders such as acne.

## SUMMARY OF THE INVENTION

The present invention provides methods and compositions for inhibiting sebum production in a subject. More particularly, the present invention provides the use of a compound that inhibits the enzyme acyl-coenzyme A:fatty alcohol acyltransferase (AFAT). In a preferred embodiment, the compound inhibits both AFAT and the enzyme acyl-coenzyme A:cholesteryl acyltransferase (ACAT). Both AFAT and ACAT are active in the sebaceous gland in the production of sebum. More particularly, the present invention provides the use of a compound selected from a class of compounds, as defined below, which inhibits AFAT, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound inhibits both AFAT and ACAT.

Dual inhibiting compounds have unexpected benefits in that they are particularly effective at inhibiting sebum production in a subject, and in treating a sebaceous gland disorder in a subject resulting from, or exacerbated by, the overproduction of sebum such as, e.g., oily skin, acne, perioral dermatitis, rosacea, seborrhea, or corticosteroid-induced acneiform lesions. The type of acne can be selected from chloracne, ciliaris acne, cystic acne, keratosa acne, vulgaris acne, senile acne, and medicinal acne, among others.

The present invention further provides a method of pharmacologically treating a sebaceous gland disorder, or a condition or disease resulting from said disorder, in a subject, comprising administering to the subject a therapeutically effective amount of a compound that inhibits AFAT, and which optionally also inhibits ACAT, or a pharmaceutically acceptable salt or solvate thereof (said compound, salt or solvate hereinafter referred to as an "active compound"). In a preferred embodiment, an active compound is a compound selected from a class of compounds, as defined below, which inhibits a mammalian, and preferably a human, AFAT, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the active compound also inhibits mammalian, and preferably human, ACAT.

The ability to inhibit both AFAT and ACAT provides a benefit in treating sebaceous gland disorders because the wax ester and cholesteryl ester products of AFAT and ACAT, respectively, form a major portion of sebum, which is secreted in excess by the sebaceous gland during episodes of seborrhea and the associated acne. Dual inhibition of AFAT and ACAT provides benefits to patients suffering from disorders characterized by excess sebum secretion that are not provided by compounds that only inhibit ACAT because such dual inhibitors can be therapeutically more effective than compounds that only inhibit ACAT.

In one embodiment, the "active compound" useful according to the methods of the present invention is a compound that is a small organic molecule having a molecular weight of <1000 amu, and comprising the core structure shown below as Formula I

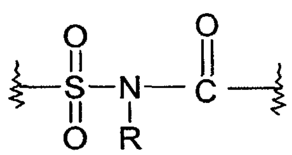

(I)

wherein R is hydrogen, a straight or branched alkyl group having from 1 to 8 carbon atom atoms or benzyl, which compound is capable of inhibiting a mammalian, and preferably a human, AFAT enzyme. In a preferred embodiment, the compound is capable of also inhibiting a mammalian, and preferably a human, ACAT enzyme.

### 1. Compounds Having the Structure of Formula II

The description provided in this section (Section 1) refers to compounds of Formula II described below. Compounds encompassed within the structure of Formula II are described in U.S. Patent No. 5,491,172 to Lee et al., issued February 13, 1996, which is incorporated by reference herein in its entirety.

In one embodiment, the "active compound" is selected from compounds having the structure of Formula II below,

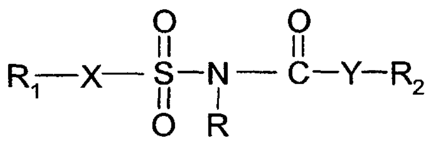

(II)

or a pharmaceutically acceptable salt or solvate thereof, wherein:

X and Y are selected from oxygen, sulfur, nitrogen and $-(CR'R'')_n-$, wherein n is an integer of from 1 to 4 and R' and R" are each independently hydrogen, alkyl, alkoxy, halogen, hydroxy, acyloxy, cycloalkyl, phenyl optionally substituted, or R' and R" together form a spirocycloalkyl or a carbonyl; with the proviso that when X and Y are both $-(CR'R'')_n-$, and R' and R" are hydrogen, and n is 1, then $R_1$ and $R_2$ are aryl;
R is hydrogen, a straight or branched alkyl of from 1 to 8 carbon atoms or benzyl;
$R_1$ and $R_2$ are each independently selected from

(a) phenyl or phenoxy, each of which is unsubstituted or substituted with 1 to 5 substituents selected from

phenyl,
an alkyl group having from 1 to 6 carbon atoms and which is straight or branched,
an alkoxy group having from 1 to 6 carbon atoms and which is straight or branched,
phenoxy,
hydroxy,
fluorine,
chlorine,
bromine,
nitro,
trifluoromethyl,
-COOH,
-COOalkyl, wherein alkyl has from 1 to 4 carbon atoms and is straight or branched, and
$-(CH_2)_pNR_3R_4$, wherein p is zero or one, and each of $R_3$ and $R_4$ is selected from hydrogen or a straight or branched alkyl group having 1 to 4 carbon atoms;

(b) 1- or 2-naphthyl, unsubstituted or substituted with from 1 to 3 substituents selected from

phenyl,
an alkyl group having from 1 to 6 carbon atoms and which is straight or branched,
an alkoxy group having from 1 to 6 carbon atoms and which is straight or branched,
hydroxy,
phenoxy,
fluorine,
chlorine,
bromine,
nitro,
trifluoromethyl,
-COOH,
-COOalkyl wherein alkyl has from 1 to 4 carbon atoms and is straight or branched, and
$-(CH_2)_pNR_3R_4$ wherein p, $R_3$ and $R_4$ have the meanings defined above;

(c) arylalkyl;
(d) a straight or branched alkyl chain having from 1 to 20 carbon atoms and which is saturated or contains from 1 to 3 double bonds; and

(e) adamantyl or a cycloalkyl group wherein the cycloalkyl moiety has from 3 to 6 carbon atoms;

with the provisos that:

(i) where X is $(CH_2)_n$, Y is oxygen, and $R_1$ is a substituted phenyl, then $R_2$ is a substituted phenyl;
(ii) where Y is oxygen, X is $(CH_2)_n$, $R_2$ is phenyl or naphthyl, then $R_1$ is not a straight or branched alkyl chain;
(iii) the following compounds of Formula II are excluded:

| X | Y | R | $R_1$ | $R_2$ |
|---|---|---|---|---|
| $CH_2$ | O | H | $(CH_2)_2CH_3$ | Ph |
| $CH_2$ | O | H | $CH_3$ | Ph |
| $CH_2$ | O | H | 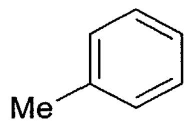 | i-Pr |

and
(iv) the compound is not [(2,4,6-triisopropyl-phenyl)-acetyl]-sulfamic acid 2,6-diisopropyl-phenyl ester or a pharmaceutically acceptable salt or solvate thereof.

In one embodiment, the compound is a compound of Formula II, wherein $R_1$ is phenyl or is phenyl disubstituted in the 2,6-positions
In another embodiment, the compound is a compound of Formula I, wherein $R_2$ is phenyl or is phenyl disubstituted in the 2,6-positions.
In another embodiment, the compound is a compound of Formula II, wherein each of $R_1$ and $R_2$ is phenyl or phenyl disubstituted in the 2,6-positions.
In another embodiment, the compound is a compound of Formula II, wherein each of $R_1$ and $R_2$ is phenyl disubstituted in the 2,6-position.
In another embodiment, the compound is a compound of Formula II, wherein $R_1$ is phenyl disubstituted in the 2,6-positions and $R_2$ is phenyl trisubstituted in the 2,4,6-positions.
In another embodiment, the compound is a compound of Formula II, wherein $R_1$ is 2,6-bis(1-methylethyl) phenyl and $R_2$ is 2,6-bis(1-methylethyl)phenyl or 2,4,6-tris(1-methylethyl)phenyl.
In another embodiment, the compound is a compound of Formula II, wherein one of $R_1$ and $R_2$ is the group

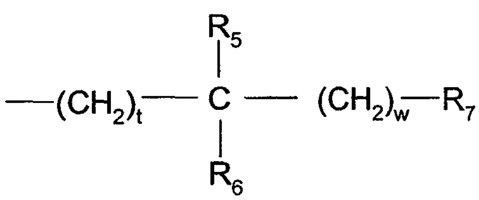

wherein t is zero or 1 to 4, w is zero or 1 to 4, with the proviso that the sum of t and w is not greater than 5; $R_5$ and $R_6$ are each independently selected from hydrogen or alkyl having from 1 to 6 carbon atoms, or when $R_5$ is hydrogen, $R_6$ can be selected from the groups defined for $R_7$; and $R_7$ is phenyl, or phenyl substituted with from 1 to 3 substituents selected from a straight or branched alkyl group having from 1 to 6 carbon atoms, straight or branched alkoxy group having from 1 to 6 carbon atoms, phenoxy, hydroxy, fluorine, chlorine, bromine, nitro, trifluoromethyl, -COOH, -COOalkyl wherein alkyl has from 1 to 4 carbon atoms, or $-(CH_2)_p\ NR_3R_4$ wherein p, $R_3$ and $R_4$ have the meanings defined above.

In another embodiment, at least one of X and Y is $-(CR'R'')_n-$.

In another embodiment, X and Y are each selected from oxygen, sulfur and $-(CR'R'')_n-$.

Other embodiments are those where the compound is a compound of Formula II wherein

X is oxygen, sulfur or $(CR'R'')_n$;
Y is oxygen, sulfur or $(CR'R'')_n$, with the proviso that at least one of X and Y is $(CR'R'')_n$ wherein n is an integer of from 1 to 4 and R' and R" are each independently hydrogen, straight or branched alkyl of from 1 to 6 carbons, optionally substituted phenyl, halogen, hydroxy, alkoxy, acyloxy, cycloalkyl, or R' and R" taken together form a carbonyl or a spirocycloalkyl group of from 3 to 10 carbons;
R is hydrogen;
$R_1$ is phenyl optionally substituted, straight or branched alkyl of from 1 to 10 carbon atoms, or cycloalkyl of from 3 to 10 carbon atoms;
$R_2$ is phenyl optionally substituted, straight or branched alkyl of from 1 to 10 carbon atoms, cycloalkyl of from 3 to 8 carbon atoms, or phenoxy optionally substituted; with the proviso that only if X is $(CR'R'')_n$ can $R_1$ be optionally substituted phenoxy and only if Y is $(CR'R'')_n$ can $R_2$ be optionally substituted phenoxy,

with the proviso that at least one of $R_1$ and $R_2$ is optionally substituted phenyl or phenoxy,
and with the further proviso that the active compound is not [(2,4,6-triisopropyl-phenyl)-acetyl]-sulfamic acid 2,6-diisopropyl-phenyl ester or a pharmaceutically acceptable salt or solvate thereof.

Other embodiments are those where the compound is a compound of Formula II wherein

X is oxygen;
Y is $(CR'R'')_n$ wherein n is an integer of from 1 to 2;
R is hydrogen;
$R_1$ is optionally substituted phenyl;
$R_2$ is optionally substituted phenyl or phenoxy, straight or branched alkyl of from 1 to 10 carbons, or cycloalkyl of from 3 to 10 carbons; and
R' and R" are each independently hydrogen, straight or branched alkyl of from 1 to 6 carbons, optionally substituted phenyl, halogen, hydroxy, alkoxy, acyloxy, cycloalkyl, or R' and R" taken together form a carbonyl or a spirocycloalkyl,
with the proviso that the active compound is not [(2,4,6-triisopropyl-phenyl)-acetyl]-sulfamic acid 2,6-diisopropyl-phenyl ester or a pharmaceutically acceptable salt or solvate thereof.

In reference to compounds of Formula II above, illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-tetradecyl, and n-octadecyl groups.

In reference to compounds of Formula II above, illustrative examples of straight or branched hydrocarbon chains having from 1 to 20 carbon atoms and having from 1 to 3 double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.

In reference to compounds of Formula II above, illustrative examples of straight or branched alkoxy groups having from 1 to 6 carbon atoms include methoxy, ethoxy, n-propoxy, t-butoxy, and pentyloxy.

In reference to compounds of Formula II above, illustrative examples of straight or branched alkyl groups having from 1 to 6 carbon atoms include methyl, ethyl, n-propyl, isopropyl, n-pentyl, n-butyl, and tert-butyl.

In reference to compounds of Formula II above, illustrative examples of cycloalkyl groups include cyclopentyl, cyclohexyl, cyclooctyl, tetrahydronaphthyl, and 1- or 2-adamantyl.

In reference to compounds of Formula II above, illustrative examples of spirocycloalkyl groups include spirocyclopropyl, spirocyclobutyl, spirocyclopentyl, and spirocyclohexyl.

In reference to compounds of Formula II above, illustrative examples of arylalkyl groups include benzyl, phenethyl, 3-phenylpropyl, 2-phenylpropyl, 4-phenylbutyl, 2-phenylbutyl, 3-phenylbutyl, benzhydryl, 2,2-diphenylethyl, and 3,3-diphenylpropyl.

Particular examples of compounds of Formula II include the following:

Sulfamic acid (phenylacetyl)-2,6-bis(1-methylethyl)phenyl ester;
Sulfamic acid[[2,6-bis(1-methylethyl)phenyl]acetyl]-2,6-bis(1-methylethyl)phenyl ester;

Sulfamic acid [[2,4,6-tris(1-methylethyl)phenyl]acetyl-2,4,6-tris(1-methylethyl)phenyl ester;
Sulfamic acid [[2,6-bis(1-methylethyl)phenyl]acetyl]-2,4,6-tris(1-methylethyl)phenyl ester;
Sulfamic acid[adamantaneacetyl]-2,6-bis[1-methylethyl)phenyl ester;
Sulfamic acid[[2,6-bis(1-methylethyl)phenyl]acetyl]-2,6-bis(1-methylethyl)phenyl ester-sodium salt;
Sulfamic acid (decanoyl)-2,6-bis-(1-methylethyl)phenyl ester;
Sulfamic acid (dodecanoyl)-2,6-bis-(1-methylethyl)phenyl ester;
2,6-Bis(1-methylethyl)-N-[[[2,4,6-tris(1-methylethyl)phenyl]methyl]sulfonyl] benzeneacetamide;
2,6-Bis(1-methylethyl)-N-[[[2,4,6-tris(1-methylethyl)phenyl]methyl]sulfonyl] benzeneacetamide-sodium salt;
2,6-Bis(1-methylethyl)phenyl[[[2,4,6-tris(1-methylethyl)phenyl]methyl]sulfonyl] carbamate;
2,6-Bis(1-methylethyl)phenyl[[[2,4,6-tris(1-methylethyl)phenyl]methyl]sulfonyl] carbamate-sodium salt;
Sulfamic acid (1-oxo-3,3-diphenylpropyl)-2,6-bis(1-methylethyl)phenyl ester;
Sulfamic acid [2,6-dichlorophenyl(acetyl)]-2,6-bis(1-methylethyl)phenyl ester;
Sulfamic acid [2,6-dichlorophenyl(acetyl)]-2,6-bis(1-methylethyl)phenyl ester;
Sulfamic acid trans-[(2-phenylcyclopropyl)-carbonyl]-2,6-bis(1-methylethyl)phenyl ester;
Sulfamic acid [2,5-dimethoxyphenyl(acetyl)]-2,6-bis(1-methylethyl)phenyl ester;
Sulfamic acid [2,4,6-trimethoxyphenyl(acetyl)]-2,6-bis(1-methylethyl)phenyl ester;
Sulfamic acid [2,4,6-trimethylphenyl(acetyl)]-2,6-bis(1-methylethyl)phenyl ester;
Sulfamic acid [2-thiophenyl(acetyl)]-2,6-bis(1-methylethyl)phenyl ester;
Sulfamic acid [3-thiophenyl(acetyl)]-2,6-bis(1-methylethyl)phenyl ester;
Sulfamic acid [2-methoxyphenyl(acetyl)]-2,6-bis(1-methylethyl)phenyl ester;
Sulfamic acid (oxophenylacetyl)-2,6-bis(1-methylethyl)phenyl ester;
Sulfamic acid [2-trifluoromethylphenyl(acetyl)]-2,6-bis(1-methylethyl)phenyl ester;
Sulfamic acid (1-oxo-2-phenylpropyl)-2,6-bis(1-methylethyl)phenyl ester;
Sulfamic acid (cyclopentylphenylacetyl)-2,6-bis(1-methylethyl)phenyl ester;
Sulfamic acid (cyclohexylacetyl)-2,6-bis(1-methylethyl)phenyl ester;
Sulfamic acid (diphenylacetyl)-2,6-bis(1-methylethyl)phenyl ester;
Sulfamic acid (triphenylacetyl)-2,6-bis(1-methylethyl)phenyl ester;
Sulfamic acid [(1-phenylcyclopentyl)carbonyl]-2,6-bis(1-methylethyl)phenyl ester;
Sulfamic acid (3-methyl-1-oxo-2-phenylpentyl)-2,6-bis(1-methylethyl)phenyl ester;
Sulfamic acid (1-oxo-2-phenylbutyl)-2,6-bis(1-methylethyl)phenyl ester;
Sulfamic acid (cyclohexylphenylacetyl)-2,6-bis(1-methylethyl)phenyl ester;
Sulfamic acid (1-oxo-2,2-diphenylpropyl)-2,6-bis(1-methylethyl)phenyl ester;
Sulfamic acid [(9H-fluoren-9-yl)carbonyl]-2,6-bis(1-methylethyl)phenyl ester;
Sulfamic acid (1-oxo-3-phenylpropyl)-2,6-bis(1-methylethyl)phenyl ester;
Sulfamic acid [1-oxo-3-[2,4,6-tris(1-methylethyl)phenyl]-2-propenyl]-2,6-bis (1-methylethyl)phenyl ester;
Sulfamic acid [1-oxo-3-[2,4,6-tris(1-methylethyl)phenyl]propyl]-2,6-bis (1-methylethyl)phenyl ester;
Sulfamic acid [(acetyloxy)[2,4,6-tris(1-methylethyl)phenyl]acetyl]-2,6-bis(1-methyl ethyl )phenyl ester;
Sulfamic acid [hydroxy[2,4,6-tris(1-methylethyl)phenyl]acetyl]-2,6-bis (1-methylethyl)phenyl ester;
Sulfamic acid [fluoro[2,4,6-tris(1methylethyl)phenyl]acetyl]-2,6-bis (1-methylethyl)phenyl ester;
Sulfamic acid (3-methyl-1-oxo-2-phenylpentyl)-2,6-bis(1-methylethyl)phenyl ester sodium salt;
Sulfamic acid [[2,4,6-tris(1-methylethyl)phenoxy]acetyl]-2,6-bis(1-methylethyl)phenyl ester;
Sulfamic acid [[2,6-bis(1-methylethyl)phenoxy]acetyl]-2,6-bis(1-methylethyl)phenyl ester;
Sulfamic acid [[2,4,6-tris(1-methylethyl)phenyl]acetyl]-2,6-bis(phenyl)phenyl ester; and the pharmaceutically acceptable salts and solvates thereof.

## 2. <u>Compounds Having the Structure of Formula III</u>

The description provided in this section (Section 2) refers to compounds of Formula III described below. Compounds encompassed within the structure of Formula III are described in U.S. Patent No. 5,214,206 to Picard et al., issued May 25, 1993, which is incorporated by reference herein in its entirety.
In another embodiment, the "active compound" is selected from compounds having the structure of Formula III below,

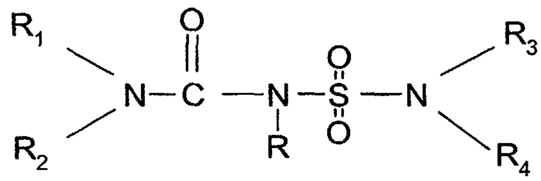

(III)

or a pharmaceutically acceptable salt or solvate thereof, wherein:

R is hydrogen, a straight or branched alkyl having from 1 to 8 carbon atoms, or benzyl; wherein each of $R_1$, $R_2$, $R_3$, and $R_4$ is selected from:

(a) hydrogen,
(b) the group

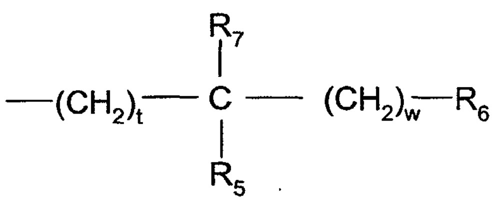

wherein t is zero to 4, and w is zero to 4, with the proviso that the sum of t and w is not greater than 5; $R_7$ and $R_5$ are independently selected from hydrogen or alkyl having from 1 to 6 carbon atoms, or when $R_7$ is hydrogen, $R_5$ can be selected from the groups defined for $R_6$; and $R_6$ is phenyl or phenyl substituted with from 1 to 3 substituents selected from straight or branched alkyl having from 1 to 6 carbon atoms, straight or branched alkoxy having from 1 to 6 carbon atoms, phenoxy, hydroxy, fluorine, chlorine, bromine, nitro, trifluoromethyl, -COOH, -COOalkyl wherein alkyl has from 1 to 4 carbon atoms, or $(CH_2)_q$-$NR_9R_8$ wherein $R_9$ and $R_8$ are independently hydrogen or alkyl of from 1 to 4 carbon atoms, and q is zero or one;
(c) a straight or branched hydrocarbon chain having from 1 to 20 carbon atoms and which is saturated or contains from 1 to 3 double bonds;
(d) an alkyl group having from 1 to 6 carbon atoms wherein the terminal carbon is substituted with hydroxy, -$NR_9R_8$ wherein $R_9$ and $R_8$ have the meanings defined above, or -COOalkyl wherein alkyl is straight or branched and has from 1 to 4 carbon atoms;
(e) -$(CH_2)_p$Q wherein p is a number from zero to 3 and Q is a 5- or 6-membered monocyclic or fused bicyclic heterocycle containing at least 1 to 4 nitrogen, oxygen, or sulfur atoms in at least one ring member;
(f) phenyl or phenyl substituted with from 1 to 3 substituents selected from straight or branched alkyl having from 1 to 6 carbon atoms, alkoxy which is straight or branched and has from 1 to 6 carbon atoms, alkylthio which is straight or branched and has from 1 to 6 carbon atoms, $(CH_2)_q$-$NR_9R_8$ wherein $R_9$ and $R_8$ and q have the meanings defined above, hydroxy, nitro, chlorine, fluorine, bromine, or trifluoromethyl; or
(g) $NR_3R_4$ taken together form a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, phenyl, benzyl, or substituted phenyl or substituted benzyl wherein the substituents vary from 1 to 3 and can be on any position of 2 through 6 of the aromatic ring and are selected from straight or branched alkyl having from 1 to 4 carbon atoms, straight or branched alkoxy having from 1 to 4 carbon atoms, hydroxy, fluorine, chlorine, bromine, trifluoromethyl, or nitro;
(h) cycloalkyl $C_{3-7}$ ; or
(i) $R_3$ is hydrogen and $R_4$ is 9-fluorenyl;

with the proviso that at least one of $R_1$ and $R_2$ and one of $R_3$ and $R_4$ is other than hydrogen
with the proviso that when both of $R_1$ and $R_2$ or when both of $R_3$ and $R_4$ are the group

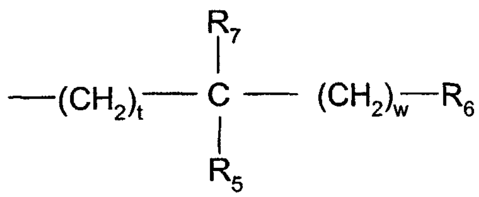

then $R_5$ is hydrogen or alkyl;

with the further provision that the compound is not [(2,4,6-triisopropyl-phenyl)-acetyl]-sulfamic acid 2,6-diisopropyl-phenyl ester or a pharmaceutically acceptable salt or solvate thereof.

In a particular embodiment, one of $R_1$ and $R_2$ is hydrogen and the other of $R_1$ and $R_2$ is phenyl or substituted phenyl.

In another embodiment, one of $R_1$ and $R_2$ is a disubstituted phenyl.

In another embodiment, one of $R_1$ and $R_2$ is a phenyl disubstituted in the 2,6-position.

In another embodiment, one of $R_3$ and $R_4$ is hydrogen and the other of $R_3$ and $R_4$ is phenyl or substituted phenyl.

In another embodiment, R is hydrogen, a straight or branched alkyl having from 1 to 8 carbon atoms, or benzyl; $R_1$ is phenyl or substituted phenyl; $R_2$ is hydrogen; and R3 and R4 are each independently hydrogen, phenyl, substituted phenyl, or a straight or branched hydrocarbon chain which is saturated or contains from 1 to 3 double bonds.

In another embodiment, when $R_1$ is hydrogen and $R_2$ is a straight or branched hydrocarbon chain having from 10 to 20 carbon atoms and which is saturated or contains from 1 to 3 double bonds, or $R_2$ is a phenyl group disubstituted in the 2,6 positions, then $-NR_3R_4$ taken together forms a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, phenyl, benzyl, or substituted phenyl or substituted benzyl wherein the substituents vary from 1 to 3 and can be on any position of 2 through 6 of the aromatic ring and are selected from straight or branched alkyl having from 1 to 4 carbon atoms, straight or branched alkoxy having from 1 to 4 carbon atoms, hydroxy, fluorine, chlorine, bromine, trifluoromethyl, or nitro.

In another embodiment, the active compound has the structure of Formula III, wherein:

R is hydrogen, straight or branched alkyl having from 1 to 8 carbon atoms or benzyl;
$R_1$ is hydrogen;
$R_2$ is 2,6-bis(1-methylethyl)phenyl;
each of $R_3$ and $R_4$ is a straight or branched chain hydrocarbon chain having from 10 to 20 carbon atoms, and which is saturated or contains from 1 to 3 double bonds, or cycloalkyl having from 3 to 7 carbon atoms.

In reference to compounds of Formula III above, illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-tetradecyl, and n-octadecyl groups.

In reference to compounds of Formula III above, illustrative examples of straight or branched hydrocarbon chains having from 1 to 20 carbon atoms and having from 1 to 3 double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.

In reference to compounds of Formula III above, illustrative examples of straight or branched alkoxy groups having from 1 to 6 carbon atoms include, for example, methoxy, ethoxy, n-propoxy, t-butoxy, and pentyloxy.

In reference to compounds of Formula III above, the term alkylthio having from 1 to 6 carbon atoms means the group $C_{1-6}$ alkyl-S-, wherein the alkyl moiety is straight or branched.

In reference to compounds of Formula III above, a 5- or 6-membered monocyclic or fused bicyclic heterocycle is a monocyclic or fused bicyclic aromatic ring containing at least 1 to 4 heteroatoms in at least one ring, such as nitrogen, oxygen, or sulfur or a combination thereof. Such a heterocyclic group includes, for example, thienyl, benzothienyl, furanyl, benzofuranyl, pyridyl, pyrimidinyl, pyridazinyl, pyrazinyl, pyrrolyl, pyrazolyl, isothiazolyl, thiazolyl, oxazolyl, isoxazolyl, triazolyl, tetrazolyl, imidazolyl, benzothiazolyl, indolyl, quinolinyl, isoquinolinyl, or N-oxides of heterocycle containing a nitrogen atom. More specifically, such a heterocycle may be a 2- or 3-thienyl ; 2- or 3-furanyl; 2-, or 3-, or 4-pyridyl or 2-, or 3-, or 4-pyridyl N-oxide; 2-, 4-, or 5-pyrimidinyl; 3- or 4 pyridazinyl; 2-pyrazinyl; 2-pyrazinyl-N-oxide; 2-or 3-pyrrolyl; 3-, 4-, or 5-pyrazolyl; 2-, 4-, or 5-thiazolyl; 3-, 4-, or 5-isoxazolyl; 2-, 4-, or 5-oxazolyl; 3-, 4-, or 5-isothiazolyl; 5-tetrazolyl; 3- or 5-(1,2,4,-)triazolyl; 4- or 5-(1,2,3-)triazolyl; 2-, 4-, or 5-imidazolyl; 2-, 3-, 4-, 5-, 6-, or 7-indolyl; 2-, 3-, 4-, 5-, 6-, 7-, or 8 -quinolinyl; 1-, 3-, 4-, 5-, 6-, 7-, or 8-isoquinolinyl; 2-, 4-, 5-, 6-, or 7-benzothiazolyl;

or 2-, 3-, 4-, 5-, 6-, or 7-benzothienyl.

Examples of particular compounds of Formula III include:

N-[2,6-bis(1-methylethyl)phenyl]-N'-[(didecylamino)sulfonyl]urea;
N-[2,6-bis(1-methylethyl)phenyl]-N'-[(didodecylamino)sulfonyl]urea;
N-[2,6-bis(1-methylethyl)phenyl]-N'-[(dicyclohexylamino)sulfonyl]urea; and the pharmaceutically acceptable salts and solvates thereof.

3. <u>Compounds Having the Structure of Formula IV</u>

The description provided in this section (Section 3) refers to compounds of Formula IV described below. Compounds encompassed within the structure of Formula IV are described in U.S. Patent No. 5,245,068 to Picard et al., issued September 14, 1993, which is incorporated by reference herein in its entirety.

In another embodiment, the "active compound" is selected from compounds having the structure of Formula IV below,

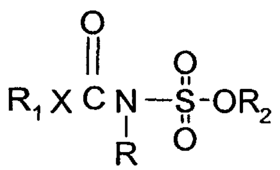

(IV)

or a pharmaceutically acceptable salt or solvate thereof, wherein:

X is oxygen or sulfur;
R is hydrogen, a straight or branched alkyl group having from 1 to 8 carbon atoms or benzyl;
each of $R_1$ and $R_2$ is selected from

(a) phenyl which is unsubstituted or is substituted with from 1 to 3 substituents selected from

phenyl,
an alkyl group having from 1 to 6 carbon atoms and which is straight or branched,
an alkoxy group having from 1 to 6 carbon atoms and which is straight or branched;
phenoxy,
hydroxy,
fluorine,
chlorine,
bromine,
trifluoromethyl,
-COOH,
-COOalkyl wherein alkyl has from 1 to 4 carbon atoms and is straight or branched,
$-(CH_2)_pNR_3R_4$ wherein p is zero or one, and each of $R_3$ and $R_4$ is selected from hydrogen or a straight or branched alkyl group having 1 to 4 carbon atoms;

(b) 1- or 2-naphthyl which is unsubstituted or is substituted with from 1 to 3 substituents selected from

phenyl,
an alkyl group having from 1 to 6 carbon atoms and which is straight or branched,
an alkoxy group having from 1 to 6 carbon atoms and which is straight or branched;
hydroxy,
phenoxy,
fluorine,
chlorine,
bromine,

nitro,
trifluoromethyl,
-COOH,
-COOalkyl wherein alkyl has from 1 to 4 carbon atoms and is straight or branched,
$-(CH_2)_pNR_3R_4$ wherein p, $R_3$ and $R_4$ have the meanings defined above;

(c) the group

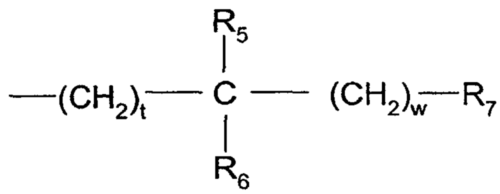

wherein t is zero or 1 to 4, and w is zero or 1 to 4; with the proviso that the sum of t and w is not greater than 5; $R_5$ and $R_6$ are independently selected from hydrogen or alkyl having from 1 to 6 carbon atoms, or when $R_5$ is hydrogen, $R_6$ can be selected from the groups defined for $R_7$ ; and $R_7$ is phenyl or phenyl substituted with from 1 to 3 substituents selected from a straight or branched alkyl group having from 1 to 6 carbon atoms, straight or branched alkoxy group having from 1 to 6 carbon atoms, phenoxy, hydroxy, fluorine, chlorine, bromine, nitro, trifluoromethyl, -COOH, -COOalkyl wherein alkyl has from 1 to 4 carbon atoms, or $-(CH_2)_p$ $NR_3R_4$ wherein p, $R_3$ and $R_4$ have the meanings defined above;
(d) $-(CH_2)_s$-Q wherein s is a number of from 0 to 3 and Q is a 5- or 6-membered monocyclic or fused bicyclic heterocycle containing at least 1 to 4 nitrogen, oxygen or sulfur atoms in at least one ring member; or
(e) a straight or branched hydrocarbon chain having from 1 to 20 carbon atoms and which is saturated or contains from 1 to 3 double bonds;
with the proviso that:

(i) one of $R_1$ or $R_2$ is phenyl or substituted phenyl and
(ii) the following compounds are excluded:

| $R_1$ | $R_2$ |
|---|---|
| $CH_3$ | Ph |
| 4-ClPh | 4-ClPh |
| i-$C_3H_7$ | 4-ClPh |
| $CH_3$ | 4-ClPh |
| 2,4,6-triClPh | $CH_3$ |
| 2,6-di-Cl-4-Ph-Ph | n-$C_3H_7$ |

In one embodiment, $R_1$ is phenyl.
In another embodiment, $R_1$ is phenyl disubstituted in the 2,6-positions.
In another embodiment, $R_2$ is phenyl.
In another embodiment, $R_2$ is phenyl disubstituted in the 2,6-positions.
In another embodiment, each of $R_1$ and $R_2$ is phenyl.
In another embodiment, each phenyl is disubstituted in the 2,6-positions.
In reference to compounds of Formula IV above, illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-tetradecyl, and n-octadecyl groups.
In reference to compounds of Formula IV above, illustrative examples of straight or branched hydrocarbon chains having from 1 to 20 carbon atoms and having from 1 to 3 double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.
In reference to compounds of Formula IV above, illustrative examples of straight or branched alkoxy groups

having from 1 to 6 carbon atoms include methoxy, ethoxy, n-propoxy, t-butoxy, and pentyloxy.

In reference to compounds of Formula IV above, illustrative examples of straight or branched alkyl groups having from 1 to 6 carbon atoms include methyl, ethyl, n-propyl, isopropyl, n-pentyl, n-butyl, and tert-butyl.

In reference to compounds of Formula IV above, a 5- or 6-membered monocyclic or fused bicyclic heterocycle is a monocyclic or fused bicyclic aromatic ring containing at least 1 to 4 heteroatoms in at least one ring, such as nitrogen, oxygen, or sulfur or a combination thereof. Such a heterocyclic group includes, for example, thienyl, benzothienyl, furanyl, benzofuranyl, pyridyl, pyrimidinyl, pyridazinyl, pyrazinyl, pyrrolyl, pyrazolyl, isothiazolyl, thiazolyl, oxazolyl, isoxazolyl, triazolyl, tetrazolyl, imidazolyl, benzothiazolyl, indolyl, quinolinyl, isoquinolinyl, or N-oxides of heterocycles containing a nitrogen atom. More specifically, such a heterocycle may be a 2- or 3-thienyl; 2- or 3-furanyl; 2-, or 3-, or 4-pyridyl or -pyridyl-N-oxide; 2-, 4-, or 5-pyrimidinyl; 3- or 4-pyridazinyl; 2-pyrazinyl; 2-pyrazinyl-N-oxide; 2- or 3-pyrrolyl; 3-, 4-, or 5-pyrazolyl; 2-, 4-, or 5-oxazolyl; 2-, 4-, or 5-thiazolyl, 3-, 4-, or 5-isoxazolyl; 3-, 4-, or 5-isothiazolyl; 5-tetrazolyl; 3- or 5-(1,2,4,-)triazolyl; 4- or 5-(1,2,3-)triazolyl; 2-, 4-, or 5-imidazolyl; 2-, 3-, 4-, 5-, 6-, or 7-indolyl; 2-, 3-, 4-, 5-, 6-, 7-, or 8-quinolinyl; 1-, 3-, 4-, 5-, 6-, 7-, or 8-isoquinolinyl; 2-, 4-, 5-, 6-, or 7-benzothiazolyl; or 2-, 3-, 4-, 5-, 6-, or 7-benzothienyl.

Examples of particular compounds of Formula IV include:

2,6-Bis(1-methylethyl)phenyl[[2,6-bis(1-methylethyl)phenoxy]sulfonyl]carbamate;
2,6-Bis(1,1-dimethylethyl)-4-methylphenyl (phenoxysulfonyl)carbamate;
2,6-Bis(1,1-dimethylethyl)-4-methylphenyl[(hexyloxy)sulfonyl]carbamate;
2,6-Bis(1,1-dimethylethyl)-4-methylphenyl[(dodecyloxy-sulfonyl]carbamate;
2,6-Bis(1-methylethyl)phenyl[(hexyloxy)sulfonyl]carbamate;
2,6-Bis(1-methylethyl)phenyl](dodecyloxy)sulfonyl]carbamate;
2,6-Bis(1,1-dimethylethyl)phenyl[[2,6-bis(1-methylethyl)phenoxy]sulfonyl]carbamate;
2,6-bis(1-methylethyl)phenyl[[2,6-bis(1-methylethyl)phenoxy]sulfonyl]carbamate, monosodium salt;

and the pharmaceutically acceptable salts and solvates thereof.

### 4. Compounds Having the Structure of Formula V

The description provided in this section (Section 4) refers to compounds of Formula V described below. Compounds encompassed within the structure of Formula V are described in U.S. Patent No. 5,254,715 to Picard et al., issued October 19, 1993, which is incorporated by reference herein in its entirety.

In one embodiment, the "active compound" is selected from compounds having the structure of Formula V below,

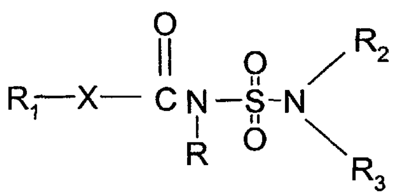

(V)

or a pharmaceutically acceptable salt or solvate thereof, wherein:

X is sulfur or oxygen;
R is hydrogen, a straight or branched alkyl having from 1 to 8 carbon atoms, or benzyl;
$R_1$ is

(a) phenyl which is unsubstituted or is substituted with from one to three substituents selected from:

phenyl,
alkyl having from one to six carbon atoms and which is straight or branched,
alkoxy having from one to six carbon atoms and which is straight or branched,
phenoxy,
hydroxy,
fluorine,

chlorine,
bromine,
nitro,
trifluoromethyl,
-COOH,
-COOalkyl wherein alkyl has from one to four carbon atoms and which is straight or branched,
$-(Ch_2)_pNR_4R_5$ wherein p is zero or one, and each of $R_4$ and $R_5$ is hydrogen or a straight or branched alkyl group having one to four carbon atoms;

(b) 1- or 2-naphthyl which is unsubstituted or substituted with one to three substituents selected from

phenyl,
alkyl having from one to six carbon atoms and which is straight or branched,
alkoxy having from one to six carbon atoms and which is straight or branched,
hydroxy,
phenoxy,
fluorine,
chlorine,
bromine,
nitro,
trifluoromethyl,
-COOH,
-COOalkyl wherein alkyl has from one to four carbon atoms and is straight or branched,
$-(CH_2)_pNR_4R_5$ wherein p, $R_4$, and $R_5$ have the meanings defined above;

(c) the group

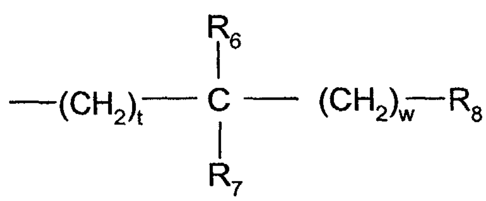

wherein t is zero or one to four and w is zero or one to four; with the proviso that the sum of t and w is not greater than five; $R_6$ and $R_7$ are independently selected from hydrogen or alkyl having for one to six carbon atoms, or when $R_6$ is hydrogen, $R_7$ can be selected from the groups defined for $R_8$ ; and $R_8$ is phenyl or phenyl substituted with from one to three substituents selected from straight or branched alkyl having from one to six carbon atoms, straight or branched alkoxy having from one to six carbon atoms, phenoxy, hydroxy, fluorine, chlorine, bromine, nitro, trifluoromethyl, -COOH, -COOalkyl wherein alkyl has from one to four carbon atoms and is straight or branched, or $-(CH_2)_pNR_4R_5$ wherein p, $R_4$ and $R_5$ have the meanings defined above;
(d) $-(CH_2)_s-Q$ wherein s is a number of from zero to three and Q is a 5- or 6-membered monocyclic or fused bicyclic heterocycle containing at least one to four nitrogen, oxygen or sulfur atoms in at least one ring member; or
(e) a straight or branched hydrocarbon chain having from 1 to 20 carbon atoms and which is saturated or contains from one to three double bonds;
each of $R_2$ and $R_3$ is

(a) hydrogen;
(b) the group

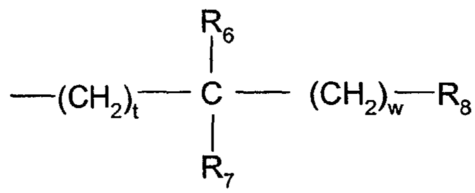

wherein t, w, $R_6$, $R_7$ and $R_8$ have the meanings defined above;
(c) a straight or branched hydrocarbon chain having from 1 to 20 carbon atoms and which is saturated or contains from one to three double bonds;
(d) an alkyl group having from one to six carbon atoms wherein the terminal carbon is substituted with hydroxy or $-NR_6R_7$ wherein $R_6$ and $R_7$ have the meanings defined above;
(e) $-(CH_2)_s$-Q wherein s and Q have the meanings defined above;
(f) phenyl or phenyl substituted with from one to three substituents selected from

phenyl,
alkyl having from one to six carbon atoms and which is straight or branched,
alkoxy having from one to six carbon atoms and which is straight or branched,
phenoxy,
hydroxy,
fluorine,
chlorine,
bromine,
nitro,
trifluoromethyl,
-COOH,
-COOalkyl wherein the alkyl moiety has from one to four carbon atoms and is straight or branched, or
$-(CH_2)_pNR_4R_5$ wherein p, $R_4$, and $R_5$ have the meanings defined above; or

(g) $NR_1R_2$ taken together form a monocyclic heterocyclic group selected from pyrrolidino, piperidino, morpholino, or piperazino, each of which is unsubstituted or substituted with one substituent selected from phenyl, straight or branched alkyl having from one to six carbon atoms;
with the provisos that:

(i) when each of $R_2$ and $R_3$ is the group

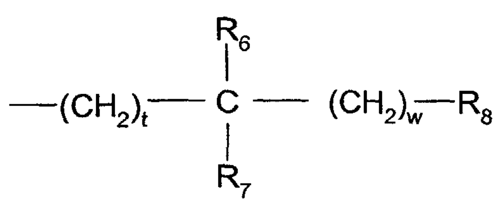

$R_7$ is hydrogen or alkyl having from one to six carbon atoms;
(ii) at least one of $R_1$, $R_2$, and $R_3$ is phenyl or substituted phenyl;
(iii) both $R_2$ and R3 are not hydrogen at the same time;
(iv) when $R_1$ is phenyl disubstituted on the 2,6-positions or trisubstituted on the 2,4,6-positions with alkyl having from one to four carbon atoms, or when $R_1$ is phenyl disubstituted on the 2,6-positions with methoxy, neither of $R_2$ or $R_3$ is phenyl; and
(v) the following compounds (wherein Ph means phenyl) are excluded:

| $R_1$ | $R_2$ | $R_3$ |
|---|---|---|
| $C_2H_5$ | H | 4-($OC_2H_5$)Ph |

(continued)

| $R_1$ | $R_2$ | $R_3$ |
|---|---|---|
| $C_2H_5$ | H | 4-(-C(=O)OC$_2$H$_5$)Ph |
| $C_2H_5$ | H | Ph |
| $CH_3$ | H | Ph |
| $CH_2$=CHCH$_2$ | H | Ph |
| $C_4H_9$ | H | Ph |
| Ph-CH$_2$- | H | $C_3H_7$ |
| 4(Cl)-Ph | H | -CH$_2$CH=CH$_2$ |
| i-C$_3$H$_7$ | H | Ph |
| Ph | $CH_3$ | $CH_3$ |
| Ph | H | Ph |
| Ph | H | n-C$_3$H$_7$ |
| Ph | H | n-C$_4$H$_9$ |
| Ph | H | 4-(-C(=O)OC$_2$H$_5$)Ph |

In one embodiment, a compound of Formula VI is a compound wherein one of $R_1$, $R_2$, and $R_3$ is phenyl disubstituted in the 2,6-positions.
In another embodiment, $R_1$ and $R_2$ are 2,6-disubstituted phenyl and $R_3$ is hydrogen.
In another embodiment, $R_1$ is 2,6-disubstituted phenyl, and $R_2$ and $R_3$ are a straight or branched hydrocarbon chain having from 1 to 20 carbon atoms, and more preferably, 6 to 18 carbon atoms, and which is saturated or contains from 1 to 3 double bonds.
In reference to compounds of Formula V above, illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-tetradecyl, and n-octadecyl groups.
In reference to compounds of Formula V above, illustrative examples of straight or branched hydrocarbon chains having from 1 to 20 carbon atoms and having from 1 to 3 double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.
In reference to compounds of Formula V above, illustrative examples of straight or branched alkoxy groups having one to six carbon atoms include methoxy, ethoxy, n-propoxy, t-butoxy, and pentyloxy.
In reference to compounds of Formula V above, illustrative examples of straight or branched alkyl groups having from one to six carbon atoms include methyl, ethyl, n-propyl, isopropyl, n-butyl, n-pentyl, n-hexyl, and tert-butyl.
In reference to compounds of Formula V above, a 5- or 6-membered monocyclic or fused bicyclic heterocycle is a monocyclic or fused bicyclic aromatic ring containing at least one to four heteroatoms in at least one ring, such as nitrogen, oxygen, or sulfur or a combination thereof. Such a heterocyclic group includes, for example, thienyl, benzothienyl, furanyl, benzofuranyl, pyridyl, pyrimidinyl, pyridazinyl, pyrazinyl, pyrrolyl, pyrazolyl, isothiazolyl, thiazolyl, oxazolyl, isoxazolyl, triazolyl, tetrazolyl, imidazolyl, benzothiazolyl, indolyl, quinolinyl, isoquinolinyl, or N-oxides of heterocycle containing a nitrogen atom. More specifically, such a heterocycle may be a 2- or 3-thienyl; 2- or 3-furanyl; 2-, or 3-, or 4-pyridyl or 2-, or 3-, or 4-pyridyl-N-oxide; 2-, 4-, or 5-pyrimidinyl; 3- or 4-pyridazinyl; 2-pyrazinyl; 2-pyrazinyl-N-oxide; 2- or 3-pyrrolyl; 3-, 4-, or 5-pyrazolyl; 2-, 4-, or 5-thiazolyl; 3-, 4-, or 5-isoxazolyl; 2-, 4-, or 5-oxazolyl; 3-, 4-, or 5-isothiazolyl; 5-tetrazolyl; 3- or 5-(1,2,4,-)triazolyl; 4-or 5-(1,2,3-)triazolyl; 2-, 4-, or 5-imidazolyl; 2-, 3-, 4-, 5-, 6-, or 7-indolyl; 2-, 3-, 4-, 5-, 6-, 7-, or 8-quinolinyl; 1-, 3-, 4-, 5-, 6-, 7-, or 8-isoquinolinyl; 2-, 4-, 5-, 6-, or 7-benzothiazolyl; or 2-, 3-, 4-, 5-, 6-, or 7-benzothienyl.
Examples of particular compounds of Formula V include:

2,6-Bis(1,1-dimethylethyl)-4-methoxyphenyl[[(2,2-diphenylethyl)amino]sulfonyl] carbamate;
2,6-Bis(1,1-dimethylethyl)-4-methoxy phenyl [[[2,6-bis(1-methylethyl)phenyl]amino] sulfonyl]carbamate;
2,6-Bis(1,1-dimethylethyl)phenyl[[(diphenylmethyl)amino]sulfonyl]carbamate;
2,6-Bis(1,1-dimethylethyl)phenyl [[[2,6-bis(1-methylethyl)phenyl]amino]sulfonyl] carbamate;
2,6-Bis(1,1-dimethylethyl)-phenyl [[(2,2-diphenylethyl)amino]sulfonyl]carbamate;
2,6-Bis(1,1-dimethylethyl)phenyl [[bis(phenylmethyl)amino]sulfonyl]carbamate;
2,6-bis(1-methylethyl)phenyl[(diphenylamino)sulfonyl]carbamate;
2,6-Bis(1-methylethyl)phenyl[(dibutylamino)sulfonyl]carbamate;

2,6-Bis(1-methylethyl)phenyl[[bis(phenylmethyl)amino]sulfonyl]carbamate;
2,6-Bis(1-methylethyl)phenyl[(1H-benzimidazol-2-ylamino)sulfonyl]carbamate;
2,6-Bis(1-methylethyl)phenyl[[2,2-diphenylethyl)amino]sulfonyl]carbamate;
2,6-Bis(1-methylethyl)phenyl[[[2,6-bis(1-methylethyl)phenyl]amino]sulfonyl] carbamate;
2,6-Bis(1-methylethyl)phenyl[[(diphenylmethyl)amino]sulfonyl]carbamate;
2,6-Bis(1,1-dimethylethyl)-4-methylphenyl[.kappa.(diphenylmethyl)amino[sulfonyl] carbamate;
2,6-Bis(1,1-dimethylethyl)-4-methylphenyl[[[bis(2,6-bis(1-methylethyl)phenyl] amino]sulfonyl]carbamate;
2,6-Bis(1,1-dimethylethyl)-4-methylphenyl[[(2,2-diphenylethyl)amino]sulfonyl] carbamate;
2,6-Bis(1,1-dimethylethyl)-4-methylphenyl[(dibutylamino)sulfonyl]carbamate;
2,6-Bis(1,1-dimethylethyl)4-methylphenyl[(dipentylamino)sulfonyl]carbamate;
2,6-Bis(1,1-dimethylethyl)-4methylphenyl[[bis(1-methylethyl)amino]sulfonyl] carbamate;
2,6-Bis(1,1-dimethylethyl)-4-methylphenyl[(dihexylamino)sulfonyl]carbamate;
2,6-Bis(1,1-dimethylethyl)-4-methylphenyl[(hexylamino)sulfonyl]carbamate;
2,6-Bis(1,1-dimethylethyl)-4-methylphenyl[[methyl(2-phenylethyl)amino]sulfonyl] carbamate;
2,6-Bis(1,1-dimethylethyl)-4-methylphenyl[.kappa.[bis-3-(dimethylamino)propyl] amino]sulfonyl]carbamate;
2,6-Bis(1,1-dimethylethyl)-4-methylphenyl[(methyl octyl amino)sulfonyl]carbamate;
2,6-Bis(1,1-dimethylethyl)-4-methyl[[bis[(tetrahydro-2-furanyl)methyl]amino] sulfonyl]carbamate;
2,6-Bis(1,1-dimethylethyl)-4-methylphenyl[(dioctylamino)sulfonyl]carbamate;
2,6-Bis(1,1-dimethylethyl)-4-methylphenyl[[[methyl 2-(2-pyridinyl)ethyl]amino]sulfonyl] carbamate, hydrochloride salt;
2,6-Bis(1,1-dimethylethyl)-4-methylphenyl[[[methyl 2-(2-pyridinyl)ethyl]amino]sulfonyl] carbamate, sodium salt;
2,6-Bis(1,1-dimethylethyl)-4-methylphenyl [(dodecylamino)sulfonyl]carbamate;
2,6-Bis(1-methylethyl)phenyl[[bis(1-methylethyl)amino]sulfonyl]carbamate;
2,6-Bis(1-methylethyl )phenyl[[(1-methylethyl)phenylmethyl)amino]sulfonyl] carbamate;
2,6-Bis(1-methylethyl)phenyl[(hexylamino)sulfonyl]carbamate;
2, 6-Bis(1-methylethyl)phenyl[(dioctylamino)sulfonyl]carbamate;
2,6-Bis(1-methylethyl)phenyl[[cyclohexyl (1-methylethyl)amino]sulfonyl]carbamate;
2,6-Bis(1-methylethyl)phenyl[(methyloctylamino)sulfonyl]carbamate;
2,6-Bis(1-methylethyl)phenyl[(dihexylamino)sulfonyl]carbamate;
2,6-Bis(1-methylethyl)phenyl ester(4-morpholinylsulfonyl)carbamic acid;
2,6-Bis(1-methylethyl)phenyl ester(1-piperldinylsulfonyl)carbamic acid;
2,6-Bis(1-methylethyl)phenyl ester(1-pyrrolidinylsulfonyl)carbamic acid;
2,6-Bis(1-methylethyl)phenyl ester, monohydrochloride[(4-methyl-1-piperazinyl) sulfonyl]carbamic acid;
2,6-Bis(1-methylethyl)-phenyl ester[(2,3-dihydro-1H-indol-1-1-yl) sulfonyl]carbamic acid;
2,6-Bis(1-methylethyl)phenyl[(dibutylamino)sulfonyl]carbamate monosodium salt; [1,1:3',1"-Terphenyl]-2'-yl[[[2,6-bis(1-methylethyl)phenyl]amino]sulfonyl]carbamate;
2,6-Bis(1,1-dimethylethyl)phenyl[[(diphenylmethyl)amino]sulfonyl]methyl carbamate; and the pharmaceutically acceptable salts and solvates thereof.

## 5. Compounds Having the Structure of Formula VI

The description provided in this section (Section 5) refers to compounds of Formula VI described below. Compounds encompassed within the structure of Formula VI are described in U.S. Patent No. 5,198,466 to Picard et al., issued March 30, 1993, which is incorporated by reference herein in its entirety.

In another embodiment, the "active compound" is selected from compounds having the structure of Formula VI below,

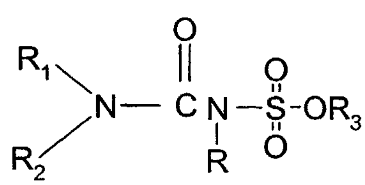

(VI)

or a pharmaceutically acceptable salt or solvate thereof, wherein:

R is hydrogen, a straight or branched alkyl having from 1 to 8 carbon atoms or benzyl; each of $R_1$ and $R_2$ is selected from

(a) hydrogen,
(b) the group

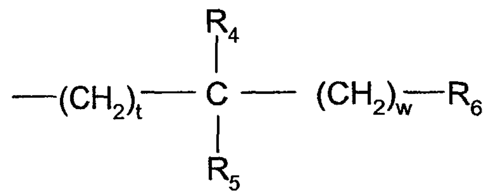

wherein t is zero to 4 and w is zero to 4; with the proviso that the sum of t and w is not greater than 5; $R_4$ and $R_5$ are independently selected from hydrogen or alkyl having from 1 to 6 carbon atoms, or when $R_4$ is hydrogen, $R_5$ can be selected from the groups defined for $R_6$; and $R_6$ is phenyl or phenyl substituted with from 1 to 3 substituents selected from straight or branched alkyl having from 1 to 6 carbon atoms, straight or branched alkoxy having from 1 to 6 carbon atoms, phenoxy, hydroxy, fluorine, chlorine, bromine, nitro, trifluoromethyl, -COOH, -COOalkyl wherein alkyl has from 1 to 4 carbon atoms, or $-(CH_2)_qNR_7R_8$ wherein $R_7$ and $R_8$ are independently hydrogen or alkyl of from 1 to 4 carbon atoms, and q is zero or one;
(c) a straight or branched hydrocarbon chain having from 1 to 20 carbon atoms and which is saturated or contains from 1 to 3 double bonds;
(d) an alkyl group having from 1 to 6 carbon atoms wherein the terminal carbon is substituted with hydroxy, $-NR_7R_8$ wherein $R_7$ and $R_8$ have the meanings defined above, or -COOalkyl wherein alkyl is straight or branched and has from 1 to 4 carbon atoms;
(e) $-(CH_2)_p$-Q wherein p is a number from zero to 3 and Q is a 5- or 6-membered monocyclic or fused bicyclic heterocycle containing at least 1 to 4 nitrogen, oxygen, or sulfur atoms in at least one ring member;
(f) phenyl or phenyl substituted with from 1 to 3 substituents selected from straight or branched alkyl having from 1 to 6 carbon atoms, alkoxy which is straight or branched and has from 1 to 6 carbon atoms, alkylthio which is straight or branched and has from 1 to 6 carbon atoms, $-(CH_2)_qNR_7R_8$ wherein $R_7$ and $R_8$ and q have the meanings defined above, hydroxy, nitro, chlorine, fluorine, bromine, or trifluoromethyl; or
(g) $NR_1R_2$ taken together form a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, phenyl, benzyl, or substituted phenyl or substituted benzyl wherein the substituents vary from 1 to 3 and can be on any position of 2 through 6 of the aromatic ring and are selected from straight or branched alkyl having from 1 to 4 carbon atoms, straight or branched alkoxy having from 1 to 4 carbon atoms, hydroxy, fluorine, chlorine, bromine, trifluoromethyl, or nitro;

$R_3$ is selected from

(a) phenyl which is unsubstituted or is substituted with from 1 to 3 substituents selected from:

phenyl,
an alkyl group having from 1 to 6 carbon atoms and which is straight or branched,
an alkoxy group having from 1 to 6 carbon atoms and which is straight or branched,
phenoxy,
hydroxy,
fluorine,
chlorine,
bromine,
nitro,
trifluoromethyl,
-COOH,
-COOalkyl wherein alkyl has from 1 to 4 carbon atoms and is straight or branched,
$-(CH_2)_sNR_9R_{10}$ wherein s is zero or one, and each of $R_9$ and
$R_{10}$ is selected from hydrogen or a straight or branched alkyl group having 1 to 4 carbon atom;

(b) 1- or 2- naphthyl which is unsubstituted or substituted with from 1 to 3 substituents selected from

phenyl,
an alkyl group having from 1 to 6 carbon atoms and which is straight or branched;
an alkoxy group having from 1 to 6 carbon atoms and which is straight or branched,
hydroxy,
phenoxy,
fluorine,
chlorine,
bromine,
nitro,
trifluoromethyl,
-COOH,
-COOalkyl wherein alkyl has from 1 to 4 carbon atoms and is straight or branched,
$-(CH_2)_sNR_9R_{10}$ wherein s, $R_9$ and $R_{10}$ have the meanings defined above;

(c) the group

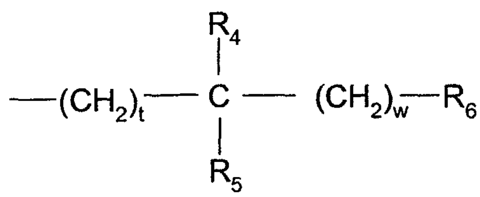

wherein t, w, $R_4$, $R_5$, and $R_6$ have the meanings defined hereinabove;
(d) $-(CH_2)_p-Q$ wherein p and Q have the meanings defined hereinabove;
(e) a straight or branched hydrocarbon chain having from 1 to 20 carbon atoms and which is saturated or contains from 1 to 3 double bonds;
with the provisos that:

(i) both $R_1$ and $R_2$ are not hydrogen at the same time;
(ii) when each of $R_1$ and $R_2$ is the group

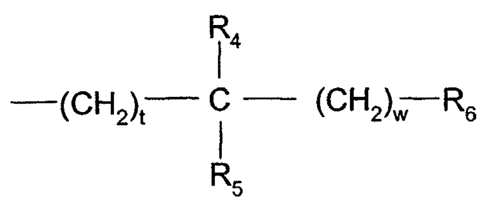

then $R_5$ is hydrogen or alkyl having from 1 to 6 carbon atoms; and
(iii) the following compounds are excluded:

| $R_1$ | $R_2$ | $R_3$ |
|---|---|---|
| Ph | H | 4-ClPh |
| Ph | H | 2,6-di$CH_3$Ph |
| Ph | H | $-CH=CH_2$ |
| n-$C_4H_9$ | H | $-CH=CH_2$ |

In one embodiment, one or $R_1$ and $R_2$ is substituted phenyl.
In another embodiment, $R_1$ is hydrogen and $R_2$ is phenyl disubstituted in the 2,6 positions.
In another embodiment, $R_1$ is hydrogen, $R_2$ is phenyl disubstituted in the 2,6 positions and $R_3$ is phenyl, substituted phenyl, or a straight or branched hydrocarbon chain having from 1 to 20 carbon atoms which is saturated

or contains from 1 to 3 double bonds.

In reference to compounds of Formula VI above, illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-ethyltetradecyl, and n-octadecyl groups.

In reference to compounds of Formula VI above, illustrative examples of straight or branched hydrocarbon chains having from 1 to 20 carbon atoms and having from 1 to 3 double bonds include ethenyl, 2-propenyl, 2-butenyl, 3-pentenyl, 2 octenyl, 5-nonenyl, 4-undecenyl, 5-heptadecenyl, 3-octadecenyl, 9-octadecenyl, 2,2-dimethyl 11 eicosenyl, 9,12-octadecadienyl, and hexadecenyl.

In reference to compounds of Formula VI above, illustrative examples of straight or branched alkoxy groups having from 1 to 6 carbon atoms include methoxy, ethoxy, n propoxy, t-butoxy, and pentyloxy.

In reference to compounds of Formula VI above, the term alkylthio having from 1 to 6 carbon atoms means the group $C_{1-6}$ alkyl-S- wherein the alkyl moiety is straight or branched.

In reference to compounds of Formula VI above, a 5- or 6-membered monocyclic or fused bicyclic heterocycle is a monocyclic or fused bicyclic aromatic ring containing at least 1 to 4 heteroatoms in at least one ring, such as nitrogen, oxygen, or sulfur or a combination thereof. Such a heterocyclic group includes, for example, thienyl, benzothienyl, furanyl, benzofuranyl, pyridyl, pyrimidinyl, pyridazinyl, pyrazinyl, pyrrolyl, pyrazolyl, isothiazolyl, oxazolyl, isoxazolyl, triazolyl, tetrazolyl, thiazolyl, imidazolyl, benzothiazolyl, indolyl, quinolinyl, isoquinolinyl, or N-oxides of heterocycles containing a nitrogen atom. More specifically, such a heterocycle may be a 2 or 3-thienyl ; 2- or 3 furanyl; 2-, or 3-, or 4 pyridyl or 2-, 3-, or 4 pyridyl-N oxide; 2-, 4 , or 5-pyrimidinyl; 3 or 4 pyridazinyl; 2 pyrazinyl; 2-pyrazinyl-N oxide; 2 or 3 pyrrolyl; 3-, 4-, or 5-pyrazolyl; 2-, 4-, or 5 thiazolyl; 3-, 4-, or 5-isoxazolyl; 2-, 4 , or 5-oxazolyl; 3-, 4-, or 5-isothiazolyl; 5 tetrazolyl; 3- or 5-(1,2,4,-)triazolyl; 4- or 5 (1,2,3-)triazolyl; 2-, 4-, or 5-imidazolyl; 2 , 3-, 4-, 5 , 6-, or 7-indolyl; 2-, 3-, 4-, 5-, 6-, 7-, or 8 quinolinyl; 1-, 3-, 4-, 5, 6-, 7-, or 8-isoquinolinyl; 2-, 4-, 5-, 6-, or 7-benzothiazolyl; or 2-, 3 , 4-, 5-, 6-, or 7 benzothienyl.

Examples of particular compounds of Formula VI include:

Hexyl[[[2,6-bis(1-methylethyl)phenyl]-amino]-carbonyl]sulfamate;
Octadecyl[[[2,6-bis(1-methylethyl)phenyl]-amino]-carbonyl]sulfamate;
Dodecyl-N-[[[2,6-bis(1-methylethyl)phenyl]-amino]-carbonyl]sulfamate;
Decyl-[[[2,6-bis(1-methylethyl)phenyl]-amino]-carbonyl]sulfamate;
(±) 1-methylheptyl [[[2,6-bis(1-methylethyl)phenyl]-amino]-carbonyl]sulfamate;
(±) 1-methylundecyl [[[2,6-bis(1-methylethyl)phenyl]-amino]-carbonyl]sulfamate;
Dodecyl-[[[2,6-bis(1-methylethyl)phenyl]-amino]-carbonyl]sulfamate, sodium salt; and the pharmaceutically acceptable salts and solvates thereof.

The present invention thus provides a method of inhibiting sebum production in a subject, comprising administering to the subject a therapeutically effective amount of a compound that inhibits AFAT, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound also inhibits ACAT. In a further preferred embodiment, the compound is an active compound of the present invention as defined above. Thus, the active compound is a compound that is a small organic molecule having a molecular weight of <1000 amu, which comprises the core structure shown below as Formula I

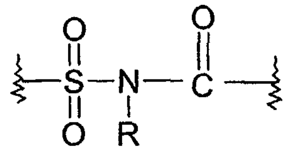

(I)

wherein R is hydrogen, a straight or branched alkyl group having from 1 to 8 carbon atom atoms or benzyl, which compound is capable of detectably inhibiting a mammalian, and preferably a human, AFAT enzyme. In a preferred embodiment, the compound is also capable of inhibiting a mammalian, and preferably a human, ACAT enzyme. Non-limiting examples of classes of compounds comprising the core structure of Formula I and from which the active compounds of the present invention can be selected include those defined according to structural formulae II through VI presented hereinabove. For all of the methods and compositions of the present invention, the active compound is not [(2,4,6-triisopropyl-phenyl)-acetyl]-sulfamic acid 2,6-diisopropyl-phenyl ester or a pharmaceutically acceptable salt or

solvate thereof. In a preferred embodiment, the active compound is administered topically.

As used herein, the term "subject" refers to any mammal that can benefit from inhibition of sebum production. In a preferred embodiment, the subject is a human. In a more preferred embodiment, the subject is a human in need of treatment to inhibit sebum production. As used herein, "a human in need of a treatment to inhibit sebum production" means a human that is suffering from overproduction of sebum as manifested by oily skin, or by the occurrence of a skin disease, disorder or condition resulting from, or exacerbated by, the overproduction of sebum, such as but not limited to acne, seborrhea, perioral dermatitis, rosacea, or corticosteroid-induced acneiform lesions. The type of acne may be selected from chloracne, ciliaris acne, cystic acne, keratosa acne, vulgaris acne, senile acne, and medicinal acne. As used herein, the term "mammalian" refers to any species of mammal, and particularly humans, as well as other primates, dogs, cats, mice, rats, and horses.

As used herein, a "therapeutically effective amount" in reference to a compound that inhibits sebum production means an amount of the compound that can detectably decrease sebum production when administered to the subject either after a single complete dose or after a complete course of treatment comprising divided doses.

As used herein, the term "AFAT' means acyl-Coenzyme A:fatty alcohol acyltransferase, and the term "ACAT" means acyl-Coenzyme A:cholesteryl acyltransferase. The term "wax ester" means an ester formed from a fatty acid and a long chain alcohol, also known as fatty alcohol. The term "cholesteryl ester" means an ester formed from a fatty acid and cholesterol.

The term "sebum" means a secretion of the sebaceous gland comprising, *inter alia,* triglycerides, free fatty acids, wax esters, squalene, cholesterol, and cholesteryl esters.

As used herein, a compound inhibits AFAT if the compound is capable of detectably inhibiting AFAT enzyme activity in any relevant in vitro enzyme assay, or in an in vivo animal model, or when administered to a subject to be treated, as compared to the enzymatic activity of those enzymes in a control experiment or in an untreated subject.

As used herein, a compound inhibits both AFAT and ACAT, i.e., is a"dual inhibitor", if the compound is capable of detectably inhibiting both AFAT and ACAT enzyme activities in any relevant in vitro enzyme assay, or in an in vivo animal model, or when administered to a subject to be treated, as compared to the enzymatic activity of those enzymes in a control experiment or in an untreated subject.

The present invention further provides a method of inhibiting the enzyme activity of AFAT in a subject, comprising administering to the subject an amount of a compound that is effective to inhibit a mammalian, and preferably a human, AFAT, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound is an active compound of the present invention encompassed within the scope of the structural formulae I through VI presented above. In a further preferred embodiment, the subject is a human. In a further preferred embodiment, the active compound is administered topically.

The present invention further provides a method of inhibiting the enzyme activity of ACAT and AFAT in a subject, comprising administering to the subject an amount of a compound that is effective to inhibit mammalian, and preferably human, AFAT and ACAT, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound is an active compound of the present invention encompassed within the scope of the structural formulae I through VI presented above. In a further preferred embodiment, the subject is a human. In a further preferred embodiment, the active compound is administered topically.

The present invention further provides a method of pharmacologically treating a sebaceous gland disorder in a subject, comprising administering to the subject a therapeutically effective amount of a compound that inhibits a mammalian, and preferably a human, AFAT, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound also inhibits mammalian, and preferably human, ACAT. In a further preferred embodiment, the compound is an active compound of the present invention encompassed within the scope of the structural formulae I through VI presented above. In a further preferred embodiment, the subject is a human. In a further preferred embodiment, the subject is a human in need of treatment of a sebaceous gland disorder. In a further preferred embodiment, the active compound is administered topically.

As used herein, a "therapeutically effective amount" in reference to a compound that can treat a sebaceous gland disorder means an amount of the compound that can detectably slow, ameliorate, reduce or reverse such a disease, disorder or condition of the skin or any symptom associated with said disease, disorder or condition, as currently affecting a subject, or that can prevent such a disease, disorder or condition, or any symptoms thereof, from occurring when administered to the subject, either after a single complete dose or after a complete course of treatment comprising divided doses has been administered.

As used herein, "a human in need of treatment of a sebaceous gland disorder" means a human that is suffering from, or is at risk of suffering from, a sebaceous gland disorder.

As used herein, the term "sebaceous gland disorder" means a disease, disorder or condition of the skin

resulting from, or exacerbated by, the overproduction of sebum, including but not limited to oily skin, acne, seborrhea, perioral dermatitis, rosacea, or corticosteroid-induced acneiform lesions. The type of acne may be selected from chloracne, ciliaris acne, cystic acne, keratosa acne, vulgaris acne, senile acne, and medicinal acne, among others.

In a preferred embodiment, the sebaceous gland disorder is oily skin.

In a further preferred embodiment, the sebaceous gland disorder is acne. In a further preferred embodiment, the acne is selected from the group consisting of chloracne, ciliaris acne, cystic acne, keratosa acne, vulgaris acne, senile acne, and medicinal acne.

In a further preferred embodiment, the sebaceous gland disorder is seborrhea.

As used herein, the terms "treat", "treating" and "treatment", and the like, as applied to sebaceous gland disorders, refer to methods that slow, ameliorate, reduce or reverse such a disease, disorder or condition of the skin or any symptoms associated with said disease, disorder or condition, as currently afflicting the subject, as well as methods that prevent such a disease, disorder or condition, or any symptoms thereof, from occurring.

As used herein, the term "pharmacological" refers to the fact that the methods of treatment of the present invention do not function only cosmetically to mask the condition, disease or disorder, or only to strip the skin of excess sebum, but are effective in treating such a condition, disease or disorder as the result of pharmacological activity. In a preferred embodiment, the pharmacological activity is manifested as inhibition of sebum production.

The present invention further provides a pharmaceutical composition comprising a therapeutically effective amount or concentration of a compound that inhibits mammalian, and preferably human, AFAT and a pharmaceutically acceptable carrier, wherein the composition is adapted for topical application, provided that the compound is not [(2,4,6-triisopropyl-phenyl)-acetyl]-sulfamic acid 2,6-diisopropyl-phenyl ester or a pharmaceutically acceptable salt or solvate thereof. In a further preferred embodiment, the compound also inhibits mammalian, and preferably human, ACAT. In a further preferred embodiment, the compound is an active compound of the present invention encompassed within the scope of the structural formulae I through VI presented above. In a non-limiting embodiment, the pharmaceutical composition is in the form of a gel, cream, lotion, paste, salve or ointment.

The present invention further provides a kit comprising a container comprising a pharmaceutical composition comprising a compound that inhibits mammalian, and preferably human, AFAT, and a pharmaceutically acceptable carrier, wherein the composition is adapted for topical application, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound also inhibits mammalian, and preferably human, ACAT. In a further preferred embodiment, the compound is an active compound of the present invention encompassed within the scope of the structural formulae I through VI presented above. The kit preferably further comprises a printed label or a set of printed instructions directing the use of the pharmaceutical composition to treat a sebaceous gland disorder, such as, e.g., oily skin, acne or seborrhea.

The present invention further provides the use of a compound that inhibits a mammalian, and preferably human, AFAT in the manufacture of a medicament for inhibiting sebum production in a subject, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound also inhibits mammalian, and preferably human, ACAT. In a further preferred embodiment, the compound is an active compound of the present invention encompassed within the scope of the structural formulae I through VI presented above. In a further preferred embodiment, the medicament is adapted for topical application.

The present invention further provides the use of a compound that inhibits a mammalian, and preferably human, AFAT in the manufacture of a medicament for treating a sebaceous gland disorder in a subject, provided that the compound is not [(2,4,6-triisopropyl-phenyl)-acetyl]-sulfamic acid 2,6-diisopropyl-phenyl ester or a pharmaceutically acceptable salt or solvate thereof. In a preferred embodiment, the compound also inhibits a mammalian, and preferably human, ACAT. In a further preferred embodiment, the compound is an active compound of the present invention encompassed within the scope of the structural formulae I through VI presented above. In a further preferred embodiment, the medicament is adapted for topical application.

## DETAILED DESCRIPTION OF THE INVENTION

The present invention thus provides methods of inhibiting sebum production and treating sebaceous gland disorders in a subject comprising administering to the subject a therapeutically effective amount of an active compound, as defined above, provided that the compound is not [(2,4,6-triisopropyl-phenyl)-acetyl]-sulfamic acid 2,6-diisopropyl-phenyl ester or a pharmaceutically acceptable salt or solvate thereof.

The active compounds of the present invention having the structure of Formula II, as defined above, can be prepared using the methods described in U.S. Patent No. 5,491,172 to Lee et al. issued February 13, 1996, and its divisional, U.S. Patent No. 5,633,287 to Lee et al., issued May 27, 1997, both of which are incorporated herein by reference in their entirety.

Active compounds of the present invention having the structure of Formula III, as defined above, can be prepared using the methods described in U.S. Patent No. 5,214,206 to Picard et al., issued May 25, 1993, which is incorporated herein by reference in its entirety.

Active compounds of the present invention having the structure of Formula IV, as defined above, can be prepared using the methods described in U.S. Patent No. 5,245,068 to Picard et al., issued September 14, 1993, which is incorporated herein by reference in its entirety.

Active compounds of the present invention having the structure of Formula V, as defined above, can be prepared using the methods described in U.S. Patent No. 5,254,715 to Picard et al., issued October 19, 1993, which is incorporated herein by reference in its entirety.

Active compounds of the present invention having the structure of Formula VI, as defined above, can be prepared using the methods described in U.S. Patent No. 5,198,466 to Picard et al., issued March 30, 1993, which is incorporated herein by reference in its entirety.

Active compounds that are small organic molecules having a molecular weight of <1000 amu, and which comprise the core structure shown above as Formula I, but which fall outside the specific structural parameters of Formulae II through VI, can be prepared by a person of ordinary skill using chemical methods analogous to those presented in the above cited patents.

The compound [(2,4,6-triisopropyl-phenyl)-acetyl]-sulfamic acid 2,6-diisopropyl-phenyl ester, also known by its generic name of "Avasimibe", is taught in U. S. Patent No. 5,491,172 and U.S. Patent No. 5,633,287 (where it is alternatively named as "sulfamic acid[[2,4,6-tris(1-methylethyl)phenyl]acetyl]-2,6-bis(1-methylethyl)phenyl ester"). Avasimibe has the structure of formula VII shown below.

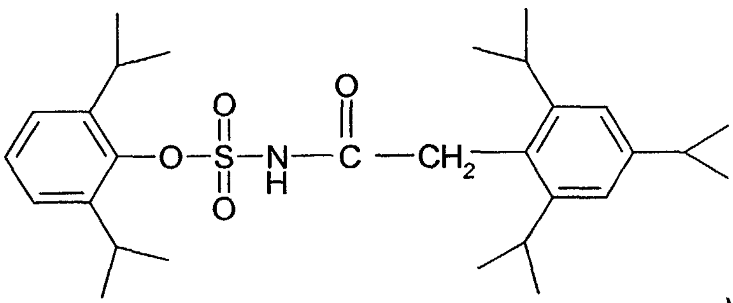

VII

Compounds of formulae I-VI as defined above that are basic in nature are capable of forming a wide variety of different salts with various inorganic and organic acids. Examples of acids that form suitable pharmaceutically acceptable salts for use in this invention are those that form non-toxic acid addition salts, i.e., salts containing pharmacologically acceptable anions such as, e.g., the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccarate, benzoate, methanesulfonate, ethanesulfonate, benzenesulfonate, p-toluenesulfonate and pamoate (i.e., 1,1'-methylene-bis-(2-hydroxy-3-napthoate)) salts.

Although such salts must be pharmaceutically acceptable for administration to animals and humans, it is often desirable in practice to initially isolate an active compound from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the basic active compounds of this invention are readily prepared by treating the basic compound with a substantially equivalent or excess amount of the chosen mineral or organic acid in an aqueous solvent medium or in a suitable organic solvent, such as methanol or ethanol, or in an aprotic solvent. Upon careful evaporation of the solvent, or upon precipitation, the desired solid salt is readily obtained.

Those active compounds that are acidic in nature are capable of forming base salts with various pharmaceutically acceptable cations. The chemical bases that are used as reagents to prepare the pharmaceutically acceptable base salts of this invention are those that form non-toxic base salts with the acidic active compounds. Such non-toxic base salts include those derived from such pharmaceutically acceptable cations as sodium, potassium, calcium and magnesium, etc. Examples of suitable amines are N,N'-dibenzyl-ethylenediamine, chloroprocaine, choline, diethanolamine, dicyclohexylamine, ethylenediamine, N-methylglucamine, and procaine (see, e.g., Berge, S.M. et al., 1977, "Pharmaceutical Salts," J. Pharm. Sci. 66:1). In one embodiment, these salts can easily be prepared by treating the corresponding acidic compounds with an aqueous solution containing the desired pharmaceutically acceptable cations, and then evaporating the resulting solution to dryness, preferably under reduced pressure. Alternatively, they can be

prepared by mixing lower alkanolic solutions of the acidic compounds and the desired alkali metal alkoxide together, and then evaporating the resulting solution to dryness, as described above. In either case, stoichiometric quantities of reagents are preferably employed in order to ensure completeness of reaction and maximum yields of the desired final products.

The compounds of the present invention can exist in different stereoisomeric forms by virtue of the presence of asymmetric centers in the compound. The present invention contemplates the use of any stereoisomeric forms of the compounds as well as mixtures thereof, including racemic mixtures.

The active compounds of the present invention can exist in unsolvated forms as well as solvated forms including hydrated forms. In general, the solvated forms, including hydrated forms, are equivalent to unsolvated forms and the present invention contemplates the use of any of them.

It will be obvious to those skilled in the art, in view of this disclosure, that the following dosage forms may comprise as the active compound either a compound of Formula I as defined above, or a corresponding pharmaceutically acceptable salt or solvate thereof.

The compounds of the present invention can be prepared and administered in a wide variety of topical, oral and parenteral dosage forms, although the compounds of the present invention are preferably adapted for topical administration.

Types of topical formulations include any that are known in the art, including gels, creams, lotions, solutions, ointments, jellies, pastes, salves and the like. Topical formulations can be prepared by combining one or more film-forming agents and the active compound in finely divided form or in solution. Film-forming agents are well known in the art and include stearyl alcohol, cetyl alcohol, propylene glycol, glycerine, carboxymethylcellulose, hydroxyethyl cellulose, and the like. These topical preparations will typically be administered directly to the skin at the site where the condition, disease, disorder, or symptom to be treated is evident. The topical preparation can also be administered to the scalp as any of the formulations listed above, or as a shampoo or scalp rinse.

Examples of vehicles for topical application of the active compounds of this invention include an aqueous or water-alcohol solution, an emulsion of the oil-in-water or water-in-oil type, an emulsified gel, or a two-phase system. Preferably, the compositions according to the invention are in the form of lotions, creams, milks, gels, masks, microspheres or nanospheres, or vesicular dispersions. In the case of vesicular dispersions, the lipids of which the vesicles are made can be of the ionic or nonionic type, or a mixture thereof. Examples of topical formulations are described in Remington's Pharmaceutical Sciences, 18th Edition, 1990, Mack Publishing Co., Easton, Pa., among other available sources.

For topical administration, an example of a gel-forming composition comprises carboxyvinyl polymers or mixtures of gel-forming compositions used in combination with an active compound. Commercially available carboxyvinyl polymers include Carbopol™ 934, 940 and 941 (Goodrich Chemicals, USA). The following excipients may be used in preparing gels for use in accordance with the invention: lower alkanols such as, for example, methanol, ethanol, isopropanol and butanol; and lower alkylene glycols having from 2 to 6 carbons such as, for example, ethylene glycol, propylene glycol and butylene glycol. Glycerine or polyethylene glycol having an average molecular weight of from 200 to 2000 can also be used in place of glycol. A preferred amount of the lower alkanol ranges from about 10% to about 50% (w/w), water ranges from about 30% to about 60% (w/w), and the polyhydric alcohol ranges from about 5% to about 40% (w/w) of the total composition.

In addition to the above-mentioned film-forming agents, various other ingredients can be incorporated into the compositions of this invention for topical administration to improve their therapeutic efficacy and stability. These include other compounds that are effective in inhibiting sebum production or in treating a sebaceous gland disorder as known in the art or to be developed in the future. The present invention contemplates the combination of two or more of the active compounds as defined above into a single therapeutic composition. Other ingredients can be included, such as astringents, soaps, detergents, surfactants, antiseptics (such as benzyl alcohol), suitable skin-permeation enhancing agents (such as diethyl sebecate) and the like, pH stabilizers, antioxidants and anti-microbial agents. These ingredients can be selected from those that are well known in the art. Topical formulations can also include other ingredients, including viscosity enhancing agents such as natural or synthetic gums, resins, methylcellulose, sodium carboxymethylcellulose, and other well-known suspending agents and emulsifiers, as well as perfumes and colorants.

Although topical administration is preferred, the active compounds of the present invention can be administered by injection, i.e., intradermally, intravenously, intramuscularly, intracutaneously, subcutaneously, intraduodenally, or intraperitoneally. The active compounds of the present invention can also be administered by inhalation, for example, intranasally, or transdermally, such as by a skin patch.

For preparing pharmaceutical compositions comprising an active compound, pharmaceutically acceptable carriers can be selected that are either solid or liquid. Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or an encapsulating material. In powders, the carrier is a finely divided solid, which is in a mixture with the

finely divided active component. In tablets, the active component is mixed with the carrier having the necessary binding properties in suitable proportions and compacted in the shape and size desired.

The powders and tablets preferably contain from five to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component, with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds and allowed to cool to a solid.

Liquid form preparations include solutions, suspensions, and emulsions, for example, water or water propylene glycol solutions. For parenteral injection, liquid preparations can be formulated in solution in aqueous polyethylene glycol solution.

Aqueous solutions suitable for oral use can be prepared by dissolving the active compound in water and adding suitable colorants, flavors, stabilizing agents, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations, which are intended to be converted shortly before use into liquid form preparations for oral administration. Such liquid forms include solutions, suspensions, and emulsions. In addition to the active compound, these preparations may contain colorants, flavors, stabilizers, buffers, artificial and natural sweeteners, dispersants, thickeners, solubilizing agents, and the like.

The pharmaceutical preparation is preferably in unit dosage form. In such form, the preparation is subdivided into unit doses containing appropriate quantities of the active compound. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

In determining what constitutes an "effective amount", a "therapeutically effective amount", a "sebum production-inhibiting amount", a "sebaceous gland disorder-inhibiting amount", an "acne-inhibiting amount", an "AFAT inhibiting amount", or an "ACAT/AFAT inhibiting amount", a number of factors will generally be considered by the attending medical practitioner in view of published clinical studies, including the subject's age, sex, weight and general condition, as well as the type and extent of the disease, disorder or condition being treated, and whether the subject is taking other medications. As such, the administered dose may fall within the ranges or concentrations recited below, or may vary outside, i.e., either below or above, those ranges depending upon the requirements of the particular subject, the severity of the condition being treated, and the particular therapeutic formulation being employed. Determination of the proper dose for a particular situation is within the skill of the art. Generally, treatment may be initiated using smaller dosages that are less than optimum for the compound. Thereafter, the dosage can be increased by small increments until the optimum effect under the circumstance is reached. For convenience, the total daily dosage can be divided and administered in portions during the day if desired.

In therapeutic use as agents for inhibiting sebum production, or for treating sebaceous gland disorders, an active compound utilized in the methods of this invention can be administered at the initial dosage of about 1 mg to about 100 mg per kilogram daily. As such, a "therapeutically effective amount", a "sebum production-inhibiting amount", a "sebaceous gland disorder-inhibiting amount", an "acne-inhibiting amount", an "AFAT inhibiting amount", or an "ACAT/AFAT inhibiting amount", of the active compound will generally range from about 0.1 mg to about 100 mg per kilogram of body weight per day. A daily dose range of about 10 mg to about 75 mg per kilogram is preferred. As used herein, the term "about" as it refers to amounts of active compound refers to the recited numerical value $\pm$ 10% of the stated value.

Alternatively, for topical applications, a "therapeutically effective amount", a "sebum production-inhibiting amount", a "sebaceous gland disorder-inhibiting amount", an "acne-inhibiting amount", an "AFAT inhibiting amount", or an "ACAT/AFAT inhibiting amount", of the active compound can be present in a topical formulation on a percentage weight basis of from about 0.1% to about 50% (w/w), more preferably from about 0.5% to about 10% (w/w), and most preferably from about 1.0% to about 5.0% (w/w).

The therapeutic formulation can be administered according to a prescribed regimen, wherein the number of applications and the duration of treatment are pre-determined by the attending medical practitioner, with adjustments in regimen made as necessary based on the subject's response to treatment. Alternatively, the subject can apply the therapeutic formulation on an as-needed basis, terminating the administration when the particular condition, disorder,

disease, or symptom thereof, subsides or otherwise reaches a satisfactory endpoint.

Examples of particular formulations comprising a compound of the present invention are provided below.

An example of the preparation of a topical gel follows.

TABLE 1

| Topical Gel: | |
|---|---|
| Ingredient | Percent by Weight |
| Active compound | 0.50 |
| Propylene glycol | 20.00 |
| Ethanol | 20.00 |
| Carboxyvinyl polymer [Carbomer 940™] | 1.00 |
| Hydroxyethyl cellulose | 0.40 |
| Benzyl alcohol | 1.00 |
| Sodium hydroxide 1N | to pH 6 |
| Distilled water | Balance |

The components other than sodium hydroxide are combined to yield a homogeneous dispersion. Addition of sodium hydroxide causes the mixture to gel yielding a ready-to-use semisolid.

An example of the preparation of a topical cream follows.

TABLE 2

| Topical Cream: | |
|---|---|
| Ingredient | Percent by Weight |
| Active compound | 0.50 |
| Stearic acid | 7.00 |
| Stearyl alcohol | 5.00 |
| Cetyl alcohol | 2.00 |
| Glycerin | 10.00 |
| Sodium laurylsulfate | 1.00 |
| Propylparaben | 0.05 |
| Methylparaben | 0.25 |
| Disodium edetate | 0.05 |
| Distilled water | Balance |

The first four ingredients are heated to approximately 70°C to produce a uniform melt. The remaining ingredients are combined, heated to approximately 75°C, and added with mixing to the previously prepared melt. The emulsion thus formed is subsequently homogenized and cooled to yield a smooth white cream.

An example of the preparation of a topical lotion follows.

TABLE 3

| Topical Lotion: | |
|---|---|
| Ingredient | Percent by Weight |
| Active compound | 0.50 |
| Glyceryl monostearate | 1.00 |
| Isopropyl palmitate | 4.00 |
| Polyethylene glycol 400 distearate | 2.00 |
| Glycerin | 10.00 |
| Methylparaben | 0.10 |
| Sodium cetylsulfate | 5.00 |
| Distilled water | Balance |

The first four ingredients are combined and heated to approximately 70°C, then added with agitation to a mixture of the remaining ingredients, also at about 70°C. The emulsion is appropriately homogenized and cooled to produce a smooth, white, pourable lotion.
An example of the preparation of a topical solution follows.

TABLE 4

| Topical Solution: | |
|---|---|
| Ingredient | Percent by Weight |
| Active compound | 0.50 |
| Propylene glycol | 20.00 |
| Ethanol | 50.00 |
| Benzyl alcohol | 1.00 |
| Disodium edetate | 0.01 |
| Propyl gallate | 0.10 |
| Citric acid | 0.20 |
| Sodium hydroxide 1N | to pH 6 |
| Distilled water | Balance |

All ingredients except sodium hydroxide are combined with agitation, and the pH of the resultant solution is adjusted with 1 N sodium hydroxide, to pH 6, to yield a free-flowing, quick-drying topical solution.
The topical formulations presented herein are examples of typical gel, cream, lotion, or solution dosage forms of active compounds for use in the treatment diseases caused by sebaceous gland disorders. Other optional components can be added or excipient ratios can be adjusted to enhance cosmetic acceptability of the formulations. Additionally, these alterations can be made to customize the composition toward a particular active compound, for example, to ensure solubilization or to enhance chemical or physical stability. Optional components would include viscosity adjusters such as celluloses, emollient oils such as mineral oil or glycerides, humectants such as polyols, cosolvents such as isopropyl alcohol or acetone, emulsifying agents of the anionic, cationic and non-ionic types, preservatives, antioxidants, opacifiers, colorants and perfumes.
An example of the preparation of an oral tablet formulation follows.

TABLE 5

| Tablet Formulation: | |
|---|---|
| Ingredient | Amount (mg) |
| Active Compound | 25 |
| Lactose | 50 |
| Cornstarch (for mix) | 10 |
| Cornstarch (paste) | 10 |
| Magnesium stearate (1%) | 5 |
| Total | 100 |

The active compound, lactose, and cornstarch (for mix) are blended to uniformity. The cornstarch (for paste) is suspended in 200 mL of water and heated with stirring to form a paste. The paste is used to granulate the mixed powders. The wet granules are passed through a No. 8 hand screen and dried at 80°C. The dry granules are lubricated with the 1% magnesium stearate and pressed into a tablet. Such tablets can be administered to a patient, such as a human from one to four times a day for treatment of sebaceous gland disorders.
An example of the preparation of an oral solution follows.

TABLE 6

| Oral Solution: | |
|---|---|
| Ingredient | Percent by Weight |
| Active Compound | 2.0 |
| Ethyl alcohol | 10.0 |

TABLE 6 (continued)

| Oral Solution: | |
|---|---|
| Ingredient | Percent by Weight |
| Benzyl alcohol | 1.0 |
| Peppermint flavor | 0.2 |
| Vanillin | 0.2 |
| Polysorbate 40 | 0.1 |
| Sucrose | 50.0 |
| Purified water | Balance |

The ingredients are combined and mixed to form a uniform solution.

The ability of a compound to inhibit AFAT, or both AFAT and ACAT, can be determined using standard assay protocols known in the art. For example, the ability of a compound to inhibit ACAT can be measured using an in vitro test cited in U.S. Patent No. 5,491,172, and more fully described in Field, F.J. and Salone R.G., 1982, Biochemica et Biophysica, 712:557-570. The assay measures the ability of a test compound to inhibit acylation of cholesterol by oleic acid by measuring the amount of radiolabeled cholesterol oleate formed using radiolabeled oleic acid in a tissue preparation containing rat liver microsomes. Alternatively, the cholesterol can be radiolabeled. An AFAT inhibition assay is described in Kolattukudy, P.E. and Rogers, L., 1986, J. Lipid Res. 27:404-411.

An example of an assay that can be used to determine whether a test compound is an inhibitor of either ACAT or AFAT, or an inhibitor of both ACAT and AFAT, is described as follows.

I. Preparation of Microsomes

Microsomes containing AFAT can be isolated from the preputial glands of mice and microsomes containing ACAT can be isolated from the liver of mice by the following procedure.

A. Preparation of Solutions

1. Wash buffer (300 mM Sucrose, 5 mM DTT) 102.7 g sucrose + 0.77 g dithiothreitol (DTT) + water to 1 L.
2. Homogenizing buffer (Wash buffer with leupeptin and ethylene glycol-bis (β-aminoethyl ether) tetra-acetic acid [EGTA]). Prepare wash buffer as above including 25 mg leupeptin and 380 mg EGTA.
3. Phosphate Buffer (0.2 M, pH 7.4) Combine 100 mL 1 M $KH_2PO_4$ with 100 mL 3 M $K_2HPO_4$ and bring to 1000 mL with water. Check pH and adjust to pH 7.4 with either 0.1 N $H_3PO_4$ or 0.1 N KOH.

B. Microsome Isolation

1. Preputial Gland (PG) or liver removal from mouse: The mice are anesthetized with ether, and the PGs or liver are removed and placed in a beaker containing wash buffer (ice cold).
2. As soon as possible, the PGs or liver are homogenized in a Potter-Elvehjem homogenizer with 15 mL of homogenizing buffer. The homogenizer is kept in a small ice bath. Work the plunger until it reaches the bottom of the tube 10 times.
3. Dilute with homogenizing buffer to a volume of 200 mL.
4. Pour homogenate into 15 × 100 mm sorvall tubes in an ice bath. Each tube holds 13.5 mL to the mark.
5. Spin in Sorvall Centrifuge at 5°C, 10,000 rpm (12,000 × G) for 15 minutes.
6. Remove the fats floating on the top with the flat blade of a spatula and decant the supernatant into fresh tubes.
7. Repeat Steps 5 and 6.
8. Carefully transfer as much of the supernatant as possible without disturbing the debris at the bottom of the tube to ultracentrifuge bottles for 50 Ti or 60 Ti Beckman Ultracentrifuge rotor. The tubes are kept in an ice bath.
9. Centrifuge in the Beckman Ultracentrifuge at 105,000 × g for 1 hour at 14°C.
10. Discard supernatant.
11. Add 1 mL of 0.2 M $KPO_4$ buffer pH 7.4 to half of the bottles in an ice bath. Scrape the whole pellet loose with a teflon rod and transfer to a 15 mL homogenizer. Wash bottle with 1 mL of buffer and transfer to one of the bottles that has a pellet but no buffer. Homogenize gently by hand.
12. Aliquot into Cryovials (Nalgene™) and store in liquid nitrogen. The microsomes remain active for at least 2 years.
13. Determine the protein concentration of the homogenate by the Lowry method. Dilute 20 µL with 180 µL

saline and assay 2 × 10 μL and 2 × 20 μL. Note: $KPO_4$ will cause a precipitate to form during the Lowry procedure (Lowry et al., 1951, J. Biol. Chem. 193:265-275). Desired protein concentration is 20 mg/mL rat or mouse PG microsomes.

C. Labeling of microsomes

One hundred microcuries of [4-$^{14}$C]cholesterol or [1-$^{14}$C]hexadecanol are dissolved in 0.1 mL 2-propanol. [4-$^{14}$C]Cholesterol-labeled microsomes (for ACAT assay) or [1-$^{14}$C]hexadecanol-labeled microsomes (for AFAT assay) are prepared by diluting a vial of stock microsomes to 4 mg protein per milliliter with Sucrose Buffer. This is followed by injection of 2.5 μL of [4-$^{14}$C]cholesterol or [1-$^{14}$C]hexadecanol, in 2-propanol, into each 1 mL of diluted microsome solution.

II. ACAT/AFAT Assay

Five microliters of test compound dissolved in dimethylsulfoxide (DMSO) is added to each of two 110 × 17 mm polypropylene conical bottom tubes. Controls and blanks receive 5 μL of DMSO alone. Each tube then receives 100 μL of 1% methyl-β-cyclodextrin in Sucrose Buffer (300 mM Sucrose, 40 mM $KH_2PO_4$, 50 mM KCl, 30 mM EDTA, pH 7.4) and 20 μL of microsomes labeled with either [4-$^{14}$C]cholesterol (for ACAT assay) or [1-$^{14}$C]hexadecanol (for AFAT assay). The assay tubes are incubated in a 37°C shaking water bath for 30 minutes. The reactions are started by adding 10 μL of 300 μM oleoyl coenzyme A in Sucrose Buffer to all tubes except the blanks, which receive only Sucrose Buffer. The reactions are stopped three minutes after oleoyl coenzyme A addition by the addition of 10 μL of 0.5% $H_2SO_4$ quench solution. Product formation is determined by transfer of 40 μL of the acidified solution to the pre-absorbent area of Whatman LK6D silica gel TLC plates, which are then dried on a warm hot plate for 5 minutes and developed in trimethylpentane/diethyl ether/acetic acid (75:25:2). The bands containing radiolabeled substrate and ester product are detected and quantitated by phosphorimaging. The fraction of ester formed is calculated from the data as E/(E + A), where E = Ester band intensity and A = Alcohol band intensity. Percent inhibition is calculated as (Xc - Xt)/Xc x 100, where Xc is the fraction ester formed in the absence of inhibitor and Xt is the fraction of ester formed in the presence of inhibitor. The concentration of inhibitor producing 50% inhibition ($IC_{50}$) can be calculated by a nonlinear least squares fit of the data to the logistic function:

$$Y = 100/[1 + (X/C)^b]$$

where Y is percent inhibition, X is the inhibitor concentration, C is the $IC_{50}$, and b is an independent fitting parameter.

For each compound, the difference between wax ester synthesis inhibition and cholesteryl ester synthesis inhibition indicates separate enzymes are responsible for wax ester synthesis and cholesteryl ester synthesis.

The ability of a compound to inhibit sebum production can be determined using standard assay protocols known in the art. For example, the ability of a compound to inhibit sebum production can be determined by visual microscopic inspection for atrophication of dermal glands in response to application of the compound to the skin. Other methods of determining sebum production in response to application of a test compound are described in U.S. Patent No. 4,224,950 to Bore et al., issued September 30, 1980; U.S. Patent No. 4,480,921 to Lévêque et al., issued November 6, 1984; U.S. Patent No. 4,981,145 to Goldstein, issued January 1, 1991; and U.S. Patent No. 6,133,326 to Mayne, issued October 17, 2000. Other methods for detecting an inhibition of sebum production are described in Japanese patent applications JP 99132712, published May 13, 1999; JP 97191066, published July 16, 1997; JP 96250184, published September 20, 1996; JP 96171643, published June 12, 1996; and JP 9675389, published March 6, 1996.

All patents, patent applications, and publications cited above are incorporated herein by reference in their entirety.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims.

## Claims

1. Use of a compound which comprises the core structure of Formula I,

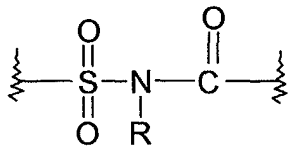

(I)

for the manufacture of a medicament for inhibiting sebum production in a subject, wherein R is hydrogen, a straight or branched alkyl group having from 1 to 8 carbon atoms or benzyl, or a pharmaceutically acceptable salt or solvate of said compound, which compound can inhibit the acyl-Coenzyme A:fatty alcohol acyltransferase activity of a mammalian AFAT enzyme;

provided that the compound is not [(2,4,6-triisopropyl-phenyl)-acetyl]-sulfamic acid 2,6-diisopropyl-phenyl ester or a pharmaceutically acceptable salt or solvate thereof.

**2.** The use according to claim 1, wherein the compound has the structure of Formula II,

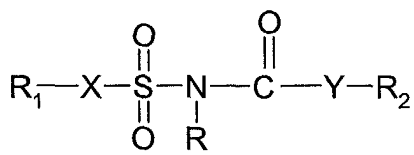

(II)

or is a pharmaceutically acceptable salt or solvate thereof, wherein:

X and Y are selected from oxygen, sulfur, nitrogen and $-(CR'R'')_n-$, wherein n is an integer of from 1 to 4 and R' and R" are each independently hydrogen, alkyl, alkoxy, halogen, hydroxy, acyloxy, cycloalkyl, phenyl optionally substituted, or R' and R" together form a spirocycloalkyl or a carbonyl; with the proviso that when X and Y are both $-(CR'R'')_n-$, and R' and R" are hydrogen, and n is 1, then $R_1$ and $R_2$ are aryl;
R is hydrogen, a straight or branched alkyl of from 1 to 8 carbon atoms or benzyl;
$R_1$ and $R_2$ are each independently selected from

(a) phenyl or phenoxy, each of which is unsubstituted or substituted with 1 to 5 substituents selected from

phenyl,
an alkyl group having from 1 to 6 carbon atoms and which is straight or branched,
an alkoxy group having from 1 to 6 carbon atoms and which is straight or branched,
phenoxy,
hydroxy,
fluorine,
chlorine,
bromine,
nitro,
trifluoromethyl,
-COOH,
-COOalkyl, wherein alkyl has from 1 to 4 carbon atoms and is straight or branched, and
$-(CH_2)_pNR_3R_4$, wherein p is zero or one, and each of $R_3$ and $R_4$ is selected from hydrogen or a straight or branched alkyl group having 1 to 4 carbon atoms;

(b) 1- or 2-naphthyl, unsubstituted or substituted with from 1 to 3 substituents selected from

phenyl,
an alkyl group having from 1 to 6 carbon atoms and which is straight or branched,
an alkoxy group having from 1 to 6 carbon atoms and which is straight or branched,

hydroxy,
phenoxy,
fluorine,
chlorine,
bromine,
nitro,
trifluoromethyl,
-COOH,
-COOalkyl wherein alkyl has from 1 to 4 carbon atoms and is straight or branched, and
$-(CH_2)_pNR_3R_4$ wherein p, $R_3$ and $R_4$ have the meanings defined above;

(c) arylalkyl;
(d) a straight or branched alkyl chain having from 1 to 20 carbon atoms and which is saturated or contains from 1 to 3 double bonds; and
(e) adamantyl or a cycloalkyl group wherein the cycloalkyl moiety has from 3 to 6 carbon atoms;

with the provisos that:
(i) where X is $(CH_2)_n$, Y is oxygen, and $R_1$ is a substituted phenyl, then $R_2$ is a substituted phenyl;
(ii) where Y is oxygen, X is $(CH_2)_n$, $R_2$ is phenyl or naphthyl, then $R_1$ is not a straight or branched alkyl chain;
(iii) the following compounds II are excluded:

| X | Y | R | $R_1$ | $R_2$ |
|---|---|---|---|---|
| $CH_2$ | O | H | $(CH_2)_2CH_3$ | Ph |
| $CH_2$ | O | H | $CH_3$ | Ph |
| $CH_2$ | O | H | 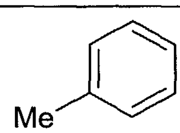 | i-Pr |

and
(iv) the compound is not [(2,4,6-triisopropyl-phenyl)-acetyl]-sulfamic acid 2,6-diisopropyl-phenyl ester or a pharmaceutically acceptable salt or solvate thereof.

**3.** The use of claim 1 or claim 2, wherein the compound is selected from the group consisting of:

Sulfamic acid (phenylacetyl)-2,6-bis(1-methylethyl)phenyl ester;
Sulfamic acid[[2,6-bis(1-methylethyl)phenyl]acetyl]-2,6-bis(1-methylethyl)phenyl ester;
Sulfamic acid [[2,4,6-tris(1-methylethyl)phenyl]acetyl-2,4,6-tris(1-methylethyl)phenyl ester;
Sulfamic acid [[2,6-bis(1-methylethyl)phenyl]acetyl]-2,4,6-tris(1-methylethyl)phenyl ester;
Sulfamic acid[adamantaneacetyl]-2,6-bis[1-methylethyl)phenyl ester;
Sulfamic acid[[2,6-bis(1-methylethyl)phenyl]acetyl]-2,6-bis(1-methylethyl)phenyl ester-sodium salt;
Sulfamic acid (decanoyl)-2,6-bis-(1-methylethyl)phenyl ester;
Sulfamic acid (dodecanoyl)-2,6-bis-(1-methylethyl)phenyl ester;
2,6-Bis(1-methylethyl)-N-[[[2,4,6-tris(1-methylethyl)phenyl]methyl]sulfonyl] benzeneacetamide;
2,6-Bis(1-methylethyl)-N-[[[2,4,6-tris(1-methylethyl)phenyl]methyl]sulfonyl] benzeneacetamide-sodium salt;
2,6-Bis(1-methylethyl)phenyl[[[2,4,6-tris(1-methylethyl)phenyl]methyl]sulfonyl] carbamate;
2,6-Bis(1-methylethyl)phenyl[[[2,4,6-tris(1-methylethyl)phenyl]methyl]sulfonyl] carbamate-sodium salt;
Sulfamic acid (1-oxo-3,3-diphenylpropyl)-2,6-bis(1-methylethyl)phenyl ester;
Sulfamic acid [2,6-dichlorophenyl(acetyl)]-2,6-bis(1-methylethyl)phenyl ester;

Sulfamic acid [2,6-dichlorophenyl(acetyl)]-2,6-bis(1-methylethyl)phenyl ester;
Sulfamic acid trans-[(2-phenylcyclopropyl)-carbonyl]-2,6-bis(1-methylethyl)phenyl ester;
Sulfamic acid [2,5-dimethoxyphenyl(acetyl)]-2,6-bis(1-methylethyl)phenyl ester;
Sulfamic acid [2,4,6-trimethoxyphenyl(acetyl)]-2,6-bis(1-methylethyl)phenyl ester;
Sulfamic acid [2,4,6-trimethylphenyl(acetyl)]-2,6-bis(1-methylethyl)phenyl ester;
Sulfamic acid [2-thiophenyl(acetyl)]-2,6-bis(1-methylethyl)phenyl ester;
Sulfamic acid [3-thiophenyl(acetyl)]-2,6-bis(1-methylethyl)phenyl ester;
Sulfamic acid [2-methoxyphenyl(acetyl)]-2,6-bis(1-methylethyl)phenyl ester;
Sulfamic acid (oxophenylacetyl)-2,6-bis(1-methylethyl)phenyl ester;
Sulfamic acid [2-trifluoromethylphenyl(acetyl)]-2,6-bis(1-methylethyl)phenyl ester;
Sulfamic acid (1-oxo-2-phenylpropyl)-2,6-bis(1-methylethyl)phenyl ester;
Sulfamic acid (cyclopentylphenylacetyl)-2,6-bis(1-methylethyl)phenyl ester;
Sulfamic acid (cyclohexylacetyl)-2,6-bis(1-methylethyl)phenyl ester;
Sulfamic acid (diphenylacetyl)-2,6-bis(1-methylethyl)phenyl ester;
Sulfamic acid (triphenylacetyl)-2,6-bis(1-methylethyl)phenyl ester;
Sulfamic acid [(1-phenylcyclopentyl)carbonyl]-2,6-bis(1-methylethyl)phenyl ester;
Sulfamic acid (3-methyl-1-oxo-2-phenylpentyl)-2,6-bis(1-methylethyl)phenyl ester;
Sulfamic acid (1-oxo-2-phenylbutyl)-2,6-bis(1-methylethyl)phenyl ester;
Sulfamic acid (cyclohexylphenylacetyl)-2,6-bis(1-methylethyl)phenyl ester;
Sulfamic acid (1-oxo-2,2-diphenylpropyl)-2,6-bis(1-methylethyl)phenyl ester;
Sulfamic acid [(9H-fluoren-9-yl)carbonyl]-2,6-bis(1-methylethyl)phenyl ester;
Sulfamic acid (1-oxo-3-phenylpropyl)-2,6-bis(1-methylethyl)phenyl ester;
Sulfamic acid [1-oxo-3-[2,4,6-tris(1-methylethyl)phenyl]-2-propenyl]-2,6-bis (1-methylethyl)phenyl ester;
Sulfamic acid [1-oxo-3-[2,4,6-tris(1-methylethyl)phenyl]propyl]-2,6-bis (1-methylethyl)phenyl ester;
Sulfamic acid [(acetyloxy)[2,4,6-tris(1-methylethyl)phenyl]acetyl]-2,6-bis(1-methyl ethyl )phenyl ester;
Sulfamic acid [hydroxy[2,4,6-tris(1-methylethyl)phenyl]acetyl]-2,6-bis (1-methylethyl)phenyl ester;
Sulfamic acid [fluoro[2,4,6-tris(1-methylethyl)phenyl]acetyl]-2,6-bis(1-methylethyl)phenyl ester;
Sulfamic acid (3-methyl-1-oxo-2-phenylpentyl)-2,6-bis(1-methylethyl)phenyl ester sodium salt;
Sulfamic acid [[2,4,6-tris(1-methylethyl)phenoxy]acetyl]-2,6-bis(1-methylethyl)phenyl ester;
Sulfamic acid [[2,6-bis(1-methylethyl)phenoxy]acetyl]-2,6-bis(1-methylethyl)phenyl ester;
Sulfamic acid [[2,4,6-tris(1-methylethyl)phenyl]acetyl]-2,6-bis(phenyl)phenyl ester; and the pharmaceutically acceptable salts and solvates thereof.

**4.** The use of claim 1, wherein the compound has the structure of Formula III,

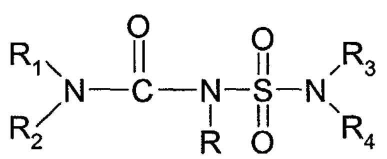

**(III)**

or is a pharmaceutically acceptable salt or solvate thereof, wherein:

R is hydrogen, a straight or branched alkyl having from 1 to 8 carbon atoms, or benzyl; wherein each of $R_1$, $R_2$, $R_3$ and $R_4$ is selected from:

(a) hydrogen,
(b) the group

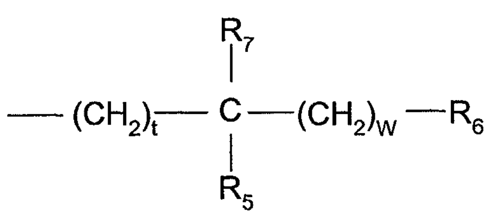

wherein t is zero to 4, and w is zero to 4, with the proviso that the sum of t and w is not greater than 5; $R_7$ and $R_5$ are independently selected from hydrogen or alkyl having from 1 to 6 carbon atoms, or when $R_7$ is hydrogen, $R_5$ can be selected from the groups defined for $R_6$; and $R_6$ is phenyl or phenyl substituted with from 1 to 3 substituents selected from straight or branched alkyl having from 1 to 6 carbon atoms, straight or branched alkoxy having from 1 to 6 carbon atoms, phenoxy, hydroxy, fluorine, chlorine, bromine, nitro, trifluoromethyl, -COOH, -COOalkyl wherein alkyl has from 1 to 4 carbon atoms, or $(CH_2)_q$-$NR_9R_8$ wherein $R_9$ and $R_8$ are independently hydrogen or alkyl of from 1 to 4 carbon atoms, and q is zero or one;
(c) a straight or branched hydrocarbon chain having from 1 to 20 carbon atoms and which is saturated or contains from 1 to 3 double bonds;
(d) an alkyl group having from 1 to 6 carbon atoms wherein the terminal carbon is substituted with hydroxy, -$NR_9R_8$ wherein $R_9$ and $R_8$ have the meanings defined above, or -COOalkyl wherein alkyl is straight or branched and has from 1 to 4 carbon atoms;
(e) -$(CH_2)_p$Q wherein p is a number from zero to 3 and Q is a 5- or 6-membered monocyclic or fused bycyclic heterocycle containing at least 1 to 4 nitrogen, oxygen, or sulfur atoms in at least one ring member;
(f) phenyl or phenyl substituted with from 1 to 3 substituents selected from straight or branched alkyl having from 1 to 6 carbon atoms, alkoxy which is straight or branched and has from 1 to 6 carbon atoms, alkylthio which is straight or branched and has from 1 to 6 carbon atoms, $(CH_2)_q$-$NR_9R_8$ wherein $R_9$ and $R_8$ and q have the meanings defined above, hydroxy, nitro, chlorine, fluorine, bromine, or trifluoromethyl; or
(g) $NR_3R_4$ taken together form a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, phenyl, benzyl, or substituted phenyl or substituted benzyl wherein the substituents vary from 1 to 3 and can be on any position of 2 through 6 of the aromatic ring and are selected from straight or branched alkyl having from 1 to 4 carbon atoms, straight or branched alkoxy having from 1 to 4 carbon atoms, hydroxy, fluorine, chlorine, bromine, trifluoromethyl, or nitro;
(h) cycloalkyl $C_{3-7}$; or
(i) $R_3$ is hydrogen and $R_4$ is 9-fluorenyl;

with the proviso that at least one of $R_1$ and $R_2$ and one of $R_3$ and $R_4$ is other than hydrogen
with the proviso that when both of $R_1$ and $R_2$ or when both of $R_3$ and $R_4$ are the group

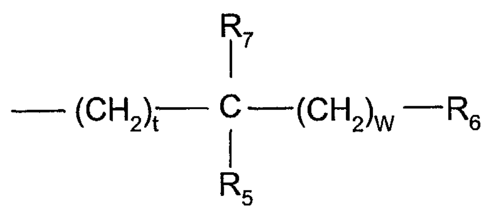

then $R_5$ is hydrogen or alkyl;
with the further proviso that the compound is not [(2,4,6-triisopropyl-phenyl)acetyl]-sulfamic acid 2,6-diisopropyl-phenyl ester or a pharmaceutically acceptable salt or solvate thereof.

**5.** The use of claim 4, wherein the compound is selected from the group consisting of

N-[2,6-bis(1-methylethyl)phenyl]-N'-[(didecylamino)sulfonyl]urea;
N-[2,6-bis(1-methylethyl)phenyl]-N'-[(didodecylamino)sulfonyl]urea;
N-[2,6-bis(1-methylethyl)phenyl]-N'-[(dicyclohexylamino)sulfonyl]urea; and the pharmaceutically acceptable salts and solvates thereof.

**6.** The use of claim 1, wherein the compound has the structure of Formula

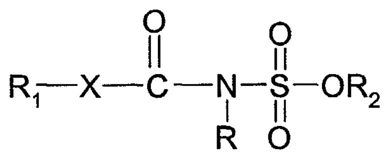 (IV)

or is a pharmaceutically acceptable salt or solvate thereof, wherein:

X is oxygen or sulfur;
R is hydrogen, a straight or branched alkyl group having from 1 to 8 carbon atoms or benzyl;

each of $R_1$ and $R_2$ is selected from

(a) phenyl which is unsubstituted or is substituted with from 1 to 3 substituents selected from

phenyl,
an alkyl group having from 1 to 6 carbon atoms and which is straight or branched,
an alkoxy group having from 1 to 6 carbon atoms and which is straight or branched,
phenoxy,
hydroxy,
fluorine,
chlorine,
bromine,
trifluoromethyl,
-COOH,
-COOalkyl wherein alkyl has from 1 to 4 carbon atoms and is straight or branched,
$-(CH_2)_pNR_3R_4$ wherein p is zero or one, and each of $R_3$ and $R_4$ is selected from hydrogen or a straight or branched alkyl group having 1 to 4 carbon atoms;

(b) 1- or 2-naphthyl which is unsubstituted or is substituted with from 1 to 3 substituents selected from

phenyl,
an alkyl group having from 1 to 6 carbon atoms and which is straight or branched,
an alkoxy group having from 1 to 6 carbon atoms and which is straight or branched;
hydroxy,
phenoxy,
fluorine,
chlorine,
bromine,
nitro,
trifluoromethyl,
-COOH,
- COOalkyl wherein alkyl has from 1 to 4 carbon atoms and is straight or branched,
$-(CH_2)_pNR_3R_4$ wherein p, $R_3$ and $R_4$ have the meanings defined above;

(c) the group

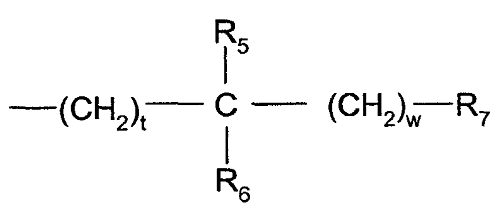

wherein t is zero or 1 to 4, and w is zero or 1 to 4; with the proviso that the sum of t and w is not greater than 5; $R_5$ and $R_6$ are independently selected from hydrogen or alkyl having from 1 to 6 carbon atoms, or when $R_5$ is hydrogen, $R_6$ can be selected from the groups defined for $R_7$ ; and $R_7$ is phenyl or phenyl substituted with from 1 to 3 substituents selected from a straight or branched alkyl group having from 1 to 6 carbon atoms, straight or branched alkoxy group having from 1 to 6 carbon atoms, phenoxy, hydroxy, fluorine, chlorine, bromine, nitro, trifluoromethyl, -COOH, -COOalkyl wherein alkyl has from 1 to 4 carbon atoms, or $-(CH_2)_p NR_3R_4$ wherein p, $R_3$ and $R_4$ have the meanings defined above;

(d) $-(CH_2)_s$-Q wherein s is a number of from 0 to 3 and Q is a 5- or 6-membered monocyclic or fused bicyclic heterocycle containing at least 1 to 4 nitrogen, oxygen or sulfur atoms in at least one ring member; or

(e) a straight or branched hydrocarbon chain having from 1 to 20 carbon atoms and which is saturated or contains from 1 to 3 double bonds;

with the proviso that:

(i) one of $R_1$ or $R_2$ is phenyl or substituted phenyl and
(ii) the following compounds are excluded:

| $R_1$ | $R_2$ |
|---|---|
| | |
| $CH_3$ | Ph |
| 4-ClPh | 4-ClPh |
| i-$C_3H_7$ | 4-ClPh |
| $CH_3$ | 4-ClPh |
| 2,4,6-triClPh | $CH_3$ |
| 2,6-di-Cl-4-Ph-Ph | n-$C_3H_7$ |

and with the further provision that the compound is not [(2,4,6-triisopropyl-phenyl)-acetyl]-sulfamic acid 2,6-diisopropyl-phenyl ester or a pharmaceutically acceptable salt or solvate thereof.

**7.** The use of claim 6 wherein the compound is selected from the group consisting of

2,6-Bis(1-methylethyl)phenyl[[2,6-bis(1-methylethyl)phenoxy]sulfonyl]carbamate;
2,6-Bis(1,1-dimethylethyl)-4-methylphenyl(phenoxysulfonyl)carbamate;
2,6-Bis(1,1-dimethylethyl)-4-methylphenyl[(hexyloxy)sulfonyl]carbamate;
2,6-Bis(1,1-dimethylethyl)-4-methyl phenyl[(dodecyloxy-sulfonyl]carbamate;
2,6-Bis(1-methylethyl)phenyl[(hexyloxy)sulfonyl]carbamate;
2,6-Bis(1-methylethyl)phenyl](dodecyloxy)sulfonyl]carbamate;
2,6-Bis(1,1-dimethylethyl)phenyl[[2,6-bis(1-methylethyl)phenoxy]sulfonyl]carbamate;
2,6-Bis(1-methylethyl)phenyl[[2,6-bis(1-methylethyl)phenoxy]sulfonyl]carbamate, monosodium salt;

and the pharmaceutically acceptable salts and solvates thereof.

**8.** The use of claim 1, wherein the compound has the structure of Formula V,

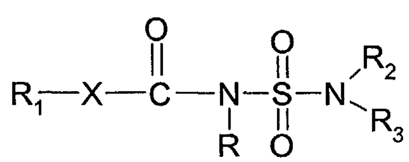
**(V)**

or is a pharmaceutically acceptable salt or solvate thereof, wherein:

X is sulfur or oxygen;
R is hydrogen, a straight or branched alkyl having from 1 to 8 carbon atoms, or benzyl;

$R_1$ is

(a) phenyl which is unsubstituted or is substituted with from one to three substituents selected from:

phenyl,
alkyl having from one to six carbon atoms and which is straight or branched,
alkoxy having from one to six carbon atoms and which is straight or branched,
phenoxy,
hydroxy,
fluorine,
chlorine,
bromine,
nitro,
trifluoromethyl,
-COOH,
-COOalkyl wherein alkyl has from one to four carbon atoms and which is straight or branched,
$-(CH_2)_pNR_4R_5$ wherein p is zero or one, and each of $R_4$ and $R_5$ is hydrogen or a straight or branched alkyl group having one to four carbon atoms;

(b) 1- or 2-naphthyl which is unsubstituted or substituted with one to three substituents selected from

phenyl,
alkyl having from one to six carbon atoms and which is straight or branched,
alkoxy having from one to six carbon atoms and which is straight or branched,
hydroxy,
phenoxy,
fluorine,
chlorine,
bromine,
nitro,
trifluoromethyl,
-COOH,
-COOalkyl wherein alkyl has from one to four carbon atoms and is straight or branched,
$-(CH_2)_pNR_4R_5$ wherein p, $R_4$, and $R_5$ have the meanings defined above;

(c) the group

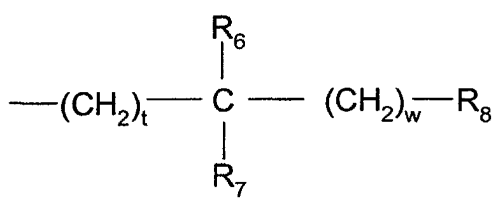

wherein t is zero or one to four and w is zero or one to four; with the proviso that the sum of t and w is not greater than five; $R_6$ and $R_7$ are independently selected from hydrogen or alkyl having for one to six carbon atoms, or when $R_6$ is hydrogen, $R_7$ can be selected from the groups defined for $R_8$ ; and $R_8$ is phenyl or phenyl substituted with from one to three substituents selected from straight or branched alkyl having from one to six carbon atoms, straight or branched alkoxy having from one to six carbon atoms, phenoxy, hydroxy, fluorine, chlorine, bromine, nitro, trifluoromethyl, -COOH, -COOalkyl wherein alkyl has from one to four carbon atoms and is straight or branched, or $-(CH_2)_pNR_4R_5$ wherein p, $R_4$ and $R_5$ have the meanings defined above;

(d) $-(CH_2)_s$-Q wherein s is a number of from zero to three and Q is a 5- or 6-membered monocyclic or fused bicyclic heterocycle containing at least one to four nitrogen, oxygen or sulfur atoms in at least one ring member; or

(e) a straight or branched hydrocarbon chain having from 1 to 20 carbon atoms and which is saturated or contains from one to three double bonds;

each of $R_2$ and $R_3$ is

(a) hydrogen;
(b) the group

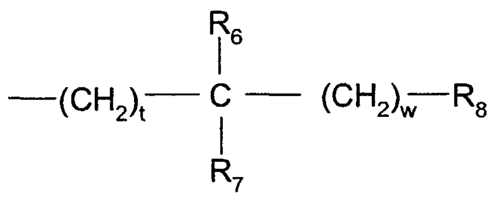

wherein t, w, $R_6$, $R_7$ and $R_8$ have the meanings defined above;
(c) a straight or branched hydrocarbon chain having from 1 to 20 carbon atoms and which is saturated or contains from one to three double bonds;
(d) an alkyl group having from one to six carbon atoms wherein the terminal carbon is substituted with hydroxy or $-NR_6R_7$ wherein $R_6$ and $R_7$ have the meanings defined above;
(e) $-(CH_2)_s-Q$ wherein s and Q have the meanings defined above;
(f) phenyl or phenyl substituted with from one to three substituents selected from

phenyl,
alkyl having from one to six carbon atoms and which is straight or branched,
alkoxy having from one to six carbon atoms and which is straight or branched,
phenoxy,
hydroxy,
fluorine,
chlorine,
bromine,
nitro,
trifluoromethyl,
-COOH,
-COOalkyl wherein the alkyl moiety has from one to four carbon atoms and is straight or branched, or
$-(CH_2)_pNR_4R_5$ wherein p, $R_4$, and $R_5$ have the meanings defined above; or

(g) $NR_1R_2$ taken together form a monocyclic heterocyclic group selected from pyrrolidino, piperidino, morpholino, or piperazino, each of which is unsubstituted or substituted with one substituent selected from phenyl, straight or branched alkyl having from one to six carbon atoms;

with the provisos that:

(i) when each of $R_2$ and $R_3$ is the group

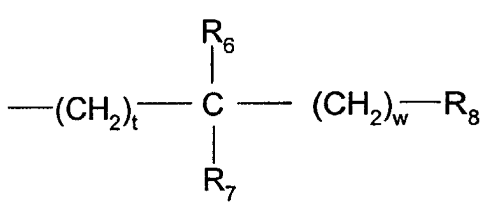

$R_7$ is hydrogen or alkyl having from one to six carbon atoms;
(ii) at least one of $R_1$, $R_2$, and $R_3$ is phenyl or substituted phenyl;
(iii) both $R_2$ and R3 are not hydrogen at the same time;
(iv) when $R_1$ is phenyl disubstituted on the 2,6-positions or trisubstituted on the 2,4,6-positions with alkyl

having from one to four carbon atoms, or when $R_1$ is phenyl disubstituted on the 2,6-positions with methoxy, neither of $R_2$ or $R_3$ is phenyl; and
(v) the following compounds (wherein Ph means phenyl) are excluded:

| $R_1$ | $R_2$ | $R_3$ |
|---|---|---|
| $C_2H_5$ | H | 4-($OC_2H_5$)Ph |
| $C_2H_5$ | H | 4-(-C(=O)$OC_2H_5$)Ph |
| $C_2H_5$ | H | Ph |
| $CH_3$ | H | Ph |
| $CH_2$=$CHCH_2$ | H | Ph |
| $C_4H_9$ | H | Ph |
| Ph-$CH_2$- | H | $C_3H_7$ |
| 4(Cl)-Ph | H | -$CH_2$CH=$CH_2$ |
| i-$C_3H_7$ | H | Ph |
| Ph | $CH_3$ | $CH_3$ |
| Ph | H | Ph |
| Ph | H | n-$C_3H_7$ |
| Ph | H | n-$C_4H_9$ |
| Ph | H | 4-(-C(=O)$OC_2H_5$)Ph |

and with the further provision that the compound is not [(2,4,6-triisopropyl-phenyl)-acetyl]-sulfamic acid 2,6-diisopropyl-phenyl ester or a pharmaceutically acceptable salt or solvate thereof.

**9.** The use of claim 8, wherein the compound is selected from the group consisting of

2,6-Bis(1,1-dimethylethyl)-4-methoxyphenyl[[(2,2-diphenylethyl)amino]sulfonyl] carbamate;
2,6-Bis(1,1-dimethylethyl)-4-methoxy phenyl [[[2,6-bis(1-methylethyl)phenyl]amino] sulfonyl]carbamate;
2,6-Bis(1,1-dimethylethyl)phenyl[[(diphenylmethyl)amino]sulfonyl]carbamate;
2,6-Bis(1,1-dimethylethyl)phenyl [[[2,6-bis(1-methylethyl)phenyl]amino]sulfonyl] carbamate;
2,6-Bis(1,1-dimethylethyl)-phenyl [[(2,2-diphenylethyl)amino]sulfonyl]carbamate;
2,6-Bis(1,1-dimethylethyl)phenyl [[bis(phenylmethyl)amino]sulfonyl]carbamate;
2,6-bis(1-methylethyl)phenyl[(diphenylamino)sulfonyl]carbamate;
2,6-Bis(1-methylethyl)phenyl[(dibutylamino)sulfonyl]carbamate;
2,6-Bis(1-methylethyl)phenyl[[bis(phenylmethyl)amino]sulfonyl]carbamate;
2,6-Bis(1-methylethyl)phenyl[(1H-benzimidazol-2-ylamino)sulfonyl]carbamate;
2,6-Bis(1-methylethyl)phenyl[[2,2-diphenylethyl)amino]sulfonyl]carbamate;
2,6-Bis(1-methylethyl)phenyl[[[2,6-bis(1-methylethyl)phenyl]amino]sulfonyl] carbamate;
2,6-Bis(1-methylethyl)phenyl[[(diphenylmethyl)amino]sulfonyl]carbamate;
2,6-Bis(1,1-dimethylethyl)-4-methylphenyl[.kappa.(diphenylmethyl)amino[sulfonyl] carbamate;
2,6-Bis(1,1-dimethylethyl)-4-methylphenyl[[[bis(2,6-bis(1-methylethyl)phenyl] amino]sulfonyl]carbamate;
2,6-Bis(1,1-dimethylethyl)-4-methylphenyl[[(2,2-diphenylethyl)amino]sulfonyl] carbamate;
2,6-Bis(1,1-dimethylethyl)-4-methylphenyl[(dibutylamino)sulfonyl]carbamate;
2,6-Bis(1,1-dimethylethyl)4-methylphenyl[(dipentylamino)sulfonyl]carbamate;
2,6-Bis(1,1-dimethylethyl)-4methylphenyl[[bis(1-methylethyl)amino]sulfonyl] carbamate;
2,6-Bis(1,1-dimethylethyl)-4-methylphenyl[(dihexylamino)sulfonyl]carbamate;
2,6-Bis(1,1-dimethylethyl)-4-methylphenyl[(hexylamino)sulfonyl]carbamate;
2,6-Bis(1,1-dimethylethyl)-4-methylphenyl[[methyl(2-phenylethyl)amino]sulfonyl] carbamate;
2,6-Bis(1,1-dimethylethyl)-4-methylphenyl[.kappa.[bis-3-(dimethylamino)propyl] amino]sulfonyl]carbamate;
2,6-Bis(1,1-dimethylethyl)-4-methylphenyl[(methyl octyl amino)sulfonyl]carbamate;
2,6-Bis(1,1-dimethylethyl)-4-methyl[[bis[(tetrahydro-2-furanyl)methyl]amino] sulfonyl]carbamate;
2,6-Bis(1,1-dimethylethyl)-4-methylphenyl[(dioctylamino)sulfonyl]carbamate;
2,6-Bis(1,1-dimethylethyl)-4-methylphenyl[[[methyl 2-(2-pyridinyl)ethyl]amino]sulfonyl] carbamate, hydrochloride salt;
2,6-Bis(1,1-dimethylethyl)-4-methylphenyl[[[methyl 2-(2-pyridinyl)ethyl]amino]sulfonyl] carbamate, sodium salt;

2,6-Bis(1,1-dimethylethyl)-4-methylphenyl[(dodecylamino)sulfonyl]carbamate;
2,6-Bis(1-methylethyl)phenyl[[bis(1-methylethyl)amino]sulfonyl]carbamate;
2,6-Bis(1-methylethyl)phenyl[[(1-methylethyl)phenylmethyl)amino]sulfonyl] carbamate;
2,6-Bis(1-methylethyl)phenyl[(hexylamino)sulfonyl]carbamate;
2, 6-Bis(1-methylethyl)phenyl[(dioctylamino)sulfonyl]carbamate;
2,6-Bis(1-methylethyl)phenyl[[cyclohexyl (1-methylethyl)amino]sufonyl]carbamate;
2,6-Bis(1-methylethyl)phenyl[(methyloctylamino)sulfonyl]carbamate;
2,6-Bis(1-methylethyl)phenyl[(dihexylamino)sulfonyl]carbamate;
2,6-Bis(1-methylethyl)phenyl ester(4-morpholinylsulfonyl)carbamic acid;
2,6-Bis(1-methylethyl)phenyl ester(1-plperldinylsulfonyl)carbamic acid;
2,6-Bis(1-methylethyl)phenyl ester(1-pyrrolidinylsulfonyl)carbamic acid;
2,6-Bis(1-methylethyl)phenyl ester, monohydrochloride[(4-methyl-1-piperazinyl) sulfonyl]carbamic acid;
2,6-Bis(1-methylethyl)-phenyl ester[(2,3-dihydro-1H-indol-1-1-yl)sulfonyl]carbamic acid;
2,6-Bis(1-methylethyl)phenyl[(dibutylamino)sulfonyl]carbamate monosodium salt; [1,1:3',1"-Terphenyl]-2'-yl[[[2,6-bis(1-methylethyl)phenyl]amino]sulfonyl]carbamate;
2,6-Bis(1,1-dimethylethyl)phenyl[[(diphenylmethyl)amino]sulfonyl]methyl carbamate;

and the pharmaceutically acceptable salts and solvates thereof.

**10.** The use of claim 1, wherein the compound has the structure of Formula VI,

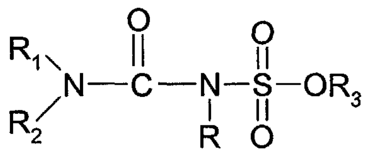

**(VI)**

or is a pharmaceutically acceptable salt or solvate thereof, wherein:

R is hydrogen, a straight or branched alkyl having from 1 to 8 carbon atoms or benzyl;
each of $R_1$ and $R_2$ is selected from

(a) hydrogen
(b) the group

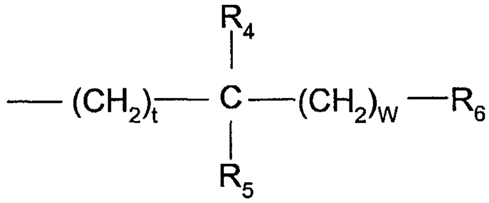

wherein t is zero to 4 and w is zero to 4; with the proviso that the sum of t and w is not greater than 5; $R_4$ and $R_5$ are independently selected from hydrogen or alkyl having from 1 to 6 carbon atoms, or when $R_4$ is hydrogen, $R_5$ can be selected from the groups defined for $R_6$; and $R_6$ is phenyl or phenyl substituted with from 1 to 3 substituents selected from straight or branched alkyl having from 1 to 6 carbon atoms, straight or branched alkoxy having from 1 to 6 carbon atoms, phenoxy, hydroxy, fluorine, chlorine, bromine, nitro, trifluoromethyl, -COOH, -COOalkyl wherein alkyl has from 1 to 4 carbon atoms, or $-(CH_2)_qNR_7R_8$ wherein $R_7$ and $R_8$ are independently hydrogen or alkyl of from 1 to 4 carbon atoms, and q is zero or one;
(c) a straight or branched hydrocarbon chain having from 1 to 20 carbon atoms and which is saturated or contains 1 to 3 double bonds;
(d) an alkyl group having from 1 to 6 carbon atoms wherein the terminal carbon is substituted with hydroxy, $-NR_7R_8$ wherein $R_7$ and $R_8$ have the meanings defined above, or -COOalkyl wherein alkyl is straight or branched and has from 1 to 4 carbon atoms;

(e) $-(CH_2)_p-Q$ wherein p is a number from zero to 3 and Q is a 5- or 6-membered monocyclic or fused bicyclic heterocycle containing at least 1 to 4 nitrogen, oxygen, or sulfur atoms in at least one ring member;
(f) phenyl or phenyl substituted with from 1 to 3 substituents selected from straight or branched alkyl having from 1 to 6 carbon atoms, alkoxy which is straight or branched and has from 1 to 6 carbon atoms, alkylthio which is straight or branched and has from 1 to 6 carbon atoms, $-(CH_2)_qNR_7R_8$ wherein $R_7$ and $R_8$ and q have the meanings defined above, hydroxy, nitro, chlorine, fluorine, bromine, or trifluoromethyl; or
(g) $NR_1R_2$ taken together form a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, phenyl, benzyl, or substituted phenyl or substituted benzyl wherein the substituents vary from 1 to 3 and can be on any position of 2 through 6 of the aromatic ring and are selected from straight or branched alkyl having from 1 to 4 carbon atoms, straight or branched alkoxy having from 1 to 4 carbon atoms, hydroxy, fluorine, chlorine, bromine, trifluoromethyl, or nitro;

$R_3$ is selected from

(a) phenyl which is unsubstituted or is substituted with from 1 to 3 substituents selected from:

phenyl,
an alkyl group having from 1 to 6 carbon atoms and which is straight or branched,
an alkoxy group having from 1 to 6 carbon atoms and which is straight or branched,
phenoxy,
hydroxy,
fluorine,
chlorine,
bromine,
nitro,
trifluoromethyl,

- COOH,
- COOalkyl wherein alkyl has from 1 to 4 carbon atoms and is straight or branched,
- $(CH_2)_sNR_9R_{10}$ wherein s is zero or one, and each of $R_9$ and $R_{10}$ is selected from hydrogen or a straight or branched alkyl group having 1 to 4 carbon atom;

(b) 1- or 2- naphthyl which is unsubstituted or substituted with from 1 to 3 substituents selected from

phenyl,
an alkyl group having from 1 to 6 carbon atoms and which is straight or branched;
an alkoxy group having from 1 to 6 carbon atoms and which is straight or branched,
hydroxy,
phenoxy,
fluorine,
chlorine,
bromine,
nitro,
trifluoromethyl,
-COOH,
-COOalkyl wherein alkyl has from 1 to 4 carbon atoms and is straight or branched,
$-(CH_2)_sNR_9R_{10}$ wherein s, $R_9$ and $R_{10}$ have the meanings defined above;

(c) the group

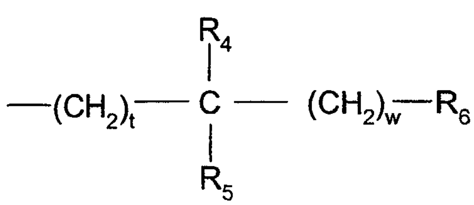

wherein t, w, $R_4$, $R_5$, and $R_6$ have the meanings defined hereinabove;
(d) $-(CH_2)_p-Q$ wherein p and Q have the meanings defined hereinabove;
(e) a straight or branched hydrocarbon chain having from 1 to 20 carbon atoms and which is saturated or contains from 1 to 3 double bonds;
with the provisos that:
(i) both $R_1$ and $R_2$ are not hydrogen at the same time;
(ii) when each of $R_1$ and $R_2$ is the group

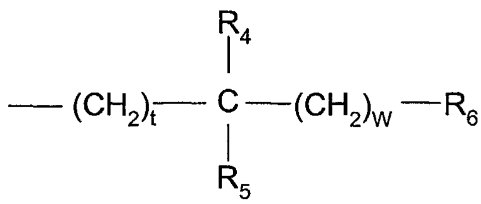

then $R_5$ is hydrogen or alkyl having from 1 to 6 carbon atoms; and
(iii) the following compounds are excluded:

| $R_1$ | $R_2$ | $R_3$ |
|---|---|---|
| Ph | H | 4-ClPh |
| Ph | H | 2,6-di$CH_3$Ph |
| Ph | H | $-CH=CH_2$ |
| n-$C_4H_9$ | H | $-CH=CH_2$ |

and with the further provision that the compound is not [(2,4,6-triisopropyl-phenyl)-acetyl]-sulfamic acid 2,6-diisopropyl-phenyl ester or a pharmaceutically acceptable salt or solvate thereof.

**11.** The use of claim 10, wherein the compound is selected from the group consisting of

Hexyl[[[2,6-bis(1-methylethyl)phenyl]-amino]-carbonyl]sulfamate;
Octadecyl[[[2,6-bis(1-methylethyl)phenyl]-amino]-carbonyl]sulfamate;
Dodecyl-N-[[[2,6-bis(1-methylethyl)phenyl]-amino]-carbonyl]sulfamte;
Decyl-[[[2,6-bis(1-methylethyl)phenyl]-amino]-carbonyl]sulfamate;
(±) 1-methylheptyl [[[2,6-bis(1-methylethyl)phenyl]-amino]-carbonyl]sulfamate;
(±) 1-methylundecyl [[[2,6-bis(1-methylethyl)phenyl]-amino]-carbonyl]sulfamate;
Dodecyl-[[[2,6-bis(1-methylethyl)phenyl]-amino]-carbonyl]sulfamate, sodium salt; and the pharmaceutically acceptable salts and solvates thereof.

**12.** The use of claim 1, wherein the mammalian AFAT enzyme is a human AFAT enzyme.

**13.** The use of claim 12, wherein the sebaceous gland disorder is selected from the group consisting of oily skin, acne, seborrhoea, perioral dermatitis, rosacea, and corticosteroid-induced acneform lesions.

**14.** A compound comprising the core structure of Formula I,

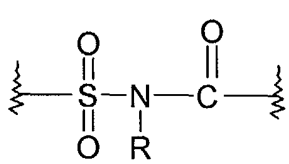

**(I)**

for use as a sebum production inhibitor in a subject, wherein R is hydrogen, a straight or branched alkyl group having from 1 to 8 carbon atoms or benzyl, or a pharmaceutically acceptable salt or solvate of said compound, which compound can inhibit the acyl-Coenzyme A:fatty alcohol acyltransferase activity of a mammalian AFAT enzyme;

provided that the compound is not [(2,4,6-triisopropyl-phenyl)-acetyl]-sulfamic acid 2,6-diisopropyl-phenyl ester or a pharmaceutically acceptable salt or solvate thereof.

**15.** A pharmaceutical composition for inhibiting sebum production comprising a therapeutically effective amount or concentration of a compound comprising the core structure of Formula I,

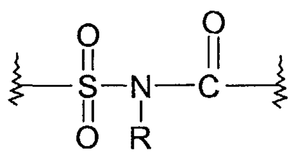

**(I)**

wherein R is hydrogen, a straight or branched alkyl group having from 1 to 8 carbon atoms or benzyl, or a pharmaceutically acceptable salt or solvate of said compound, which compound can inhibit the acyl-Coenzyme A:fatty alcohol acyltransferase activity of a mammalian AFAT enzyme, and a pharmaceutically acceptable carrier, which composition is adapted for topical application;

provided that the compound is not [(2,4,6-triisopropyl-phenyl)-acetyl]-sulfamic acid 2,6-diisopropyl-phenyl ester or a pharmaceutically acceptable salt or solvate thereof.